# EUROPÄISCHE PATENTSCHRIFT

(11) **EP 3 496 594 B1**
(45) Veröffentlichungstag und Bekanntmachung des Hinweises auf die Patenterteilung: **28.02.2024**
(21) Anmeldenummer: 17749691.6
(22) Anmeldetag: 03.08.2017
(51) Int. Cl.: A61B 5/00, A61B 5/11

(54) **SYSTEM, VERFAHREN UND COMPUTERLESBARES SPEICHERMEDIUM ZUR KÖRPERHALTUNGS- UND BEWEGUNGSREGULATION**
SYSTEM, METHOD AND COMPUTER READABLE STORAGE MEDIUM FOR POSTURE AND MOVEMENT REGULATION
SYSTÈME, PROCÉDÉ ET SUPPORT LISIBLE PAR ORDINATEUR POUR LA RÉGULATION DU MAINTIEN ET DU MOUVEMENT CORPOREL

(30) Priorität: 09.08.2016 DE 102016114766
(43) Veröffentlichungstag der Anmeldung: 19.06.2019
(73) Patentinhaber: Addasense Health GmbH, 82008 Unterhaching (DE)
(72) Erfinder: Sonntag, Peter, 81371 München (DE); Bollinger, Stephan, 80469 München (DE)
(74) Vertreter: Meissner Bolte Partnerschaft mbB
(86) Internationale Anmeldenummer: PCT/EP2017/069598
(87) Internationale Veröffentlichungsnummer: WO 2018/029064

(56) Entgegenhaltungen:
- EP-A1- 1 761 167
- WO-A1-2016/055848
- DE-A1- 10 304 238
- DE-A1- 19 911 612
- DE-A1-102010 003 871
- DE-U1-202005 015 889
- DE-U1-202014 101 266
- US-A- 4 730 625
- US-A1- 2007 249 466
- US-A1- 2014 029 767
- US-A1- 2014 330 172

## Beschreibung

Die Erfindung betrifft ein Verfahren zur Körperhaltungs- und Bewegungsregulation. Des Weiteren betrifft die Erfindung ein System zur Anwendung bei dem erfindungsgemäßen Verfahren zur Körperhaltungs- und Bewegungsregulation. Außerdem betrifft die Erfindung ein computerlesbares Speichermedium.

Personen, die unter Gehstörungen und/oder Gleichgewichtsstörungen und/oder Haltungsstörungen leiden oder aufgrund von Operationen hinsichtlich ihrer Bewegungsmöglichkeiten eingeschränkt sind, sind während des Gehens meist stark verunsichert. Diese Personen nehmen häufig Schonhaltungen ein. Dies kann unter Umständen Haltungsschäden verstärken.

Aus US 2007/249466 A1 ist ein System und ein Verfahren zur Körperhaltungs-und Bewegungsregulation bekannt, das auf der Verwendung von Kopfhörern basiert, die dem Benutzer des Systems akustische Signale hinsichtlich seiner Körperhaltung und Bewegung wiedergeben.

Es ist Aufgabe der vorliegenden Erfindung, ein weiterentwickeltes System zur Körperhaltungs- und Bewegungsregulation anzugeben, so dass zumindest oben beschriebener Personenkreis noch mehr Sicherheit beim Gehen bzw. bei der Bewegung zurückerlangt. Das System soll zur Vermeidung von Unfällen bei gangunsicheren Personen beitragen. Hinsichtlich Personen mit Beeinträchtigung des Gleichgewichtssinnes soll eine zusätzliche Sicherheit geschaffen werden. Insbesondere sollen Schonhaltungen und ein Schongang sowie falsche und ungesunde Bewegungen bzw. Bewegungsabläufe vermieden werden. Außerdem soll es mit dem System zur Körperhaltungs- und Bewegungsregulation ermöglicht werden, bei Vorliegen von lediglich einseitigen Körperhaltungsschäden und/oder Gangstörungen gezielt auf diese einseitige Störung einwirken zu können.

Des Weiteren soll es aufgrund des Systems ermöglicht werden, Trainingseinheiten bei Lage-, Positions-, Rotations- und Haltungsänderungen von Körpern oder einzelnen zu trainierenden Körperteilen bewusst und korrekt durchzuführen. Das System soll beispielsweise zur Verbesserung der Gelenkbeweglichkeit oder zur Verbesserung der Beweglichkeit bei Muskelverkürzungen oder bei Kraftlosigkeit in einzelnen Körpergliedern oder bei Bewegungseinschränkungen einzelner Gliedmaßen oder Gliedergruppen als Übungsmittel dienen. Therapieziele mit einem Therapeuten sollen aufgrund der Anwendung des erfindungsgemäßen Systems beschleunigt, vereinfacht oder intensiviert werden.

Außerdem ist es Aufgabe der vorliegenden Erfindung, ein Verfahren zur Haltungs-und Bewegungsregulation für Menschen anzugeben. Das Verfahren soll mit dem erfindungsgemäßen System durchführbar sein.

Die Lösung der Aufgabe der Erfindung erfolgt durch ein Verfahren zur Körperhaltungs- und Bewegungsregulation, gemäß der Lehre nach Anspruch 1. Des Weiteren erfolgt die Lösung der Aufgabe der Erfindung durch ein System zur Haltungs- und Bewegungsregulation, gemäß der Lehre nach Anspruch 7. Außerdem erfolgt die Lösung der Aufgabe der Erfindung durch ein computerlesbares Speichermedium gemäß der Lehre nach Anspruch 14.

Die Unteransprüche stellen mindestens zweckmäßige Ausgestaltungen und Weiterbildungen des erfindungsgemäßen Systems sowie des erfindungsgemäßen Verfahrens dar.

Das erfindungsgemäße System zur Körperhaltungs- und Bewegungsregulation kann auch als System zur Geh- und Bewegungsregulationshilfe bezeichnet werden. Da eine Bewegungsregulation auch eine Geh- bzw. Gangregulation umfasst, wird im Folgenden das System als System zur Körperhaltungs- und Bewegungsregulation bezeichnet.

Das erfindungsgemäße System zur Körperhaltungs- und Bewegungsregulation umfasst:
- mindestens eine Körper-Lagesensor-Einheit,
- mindestens eine Recheneinheit,
- mindestens eine Ausgabeeinheit zur Ausgabe von zwei stereoakustischen Signalen, wobei zwischen der Recheneinheit und der Ausgabeeinheit zur Ausgabe der stereoakustischen Signale ein Audio-Modul ausgebildet ist, wobei das Audio-Modul mindestens eine Tonerzeugungs-Einheit und mindestens eine Audio-Mixer-Einheit umfasst, wobei die Körper-Lagesensor-Einheit mindestens einen Erschütterungssensor umfasst, wobei die Ausgabeeinheit ein Kopfhörer ist.

Die Ausgabeeinheit ist zur Ausgabe (mindestens) eines stereoakustischen Signals ausgebildet.

Es ist mindestens eine Ausgabeeinheit vorgesehen. Das System weist mindestens eine Ausgabeeinheit zur Ausgabe eines akustischen Signals auf.

Die Körper-Lagesensor-Einheit umfasst einen Erschütterungssensor. Es ist möglich, dass die Körper-Lagesensor-Einheit mehrere Lagesensoren umfasst. Die Lagesensoren können gleichartig oder verschiedenartig ausgebildet sein.

Insbesondere ist es möglich, dass die Körper-Lagesensor-Einheit mehrere Sensoren umfasst, die an verschiedenen Körperteilen und/oder verschiedenen Positionen des Körpers angeordnet sind.

Das erfindungsgemäße System kann des Weiteren eine Anzeigeeinheit zur visuellen Darstellung der von der Körper-Lagesensor-Einheit ermittelten Daten aufweisen. Des Weiteren ist es möglich, dass das System eine Bedieneinheit zur Einstellung von System-Parametern aufweist. In einer besonders bevorzugten Ausführungsform der Erfindung können die Bedieneinheit und die Anzeigeeinheit in einem Modul zusammengefasst sein. Bei diesem Modul kann es sich um ein sogenanntes Interface-Elemente-Modul handeln. Das Interface-Elemente-Modul kann auch als Ein- und Ausgabemodul bezeichnet werden.

Die mindestens eine Körper-Lagesensor-Einheit und/oder die mindestens eine Recheneinheit und/oder die mindestens eine Ausgabeeinheit zur Ausgabe eines akustischen Signals und/oder mindestens eine Ausgabeeinheit zur Ausgabe eines Vibrationssignals und/oder die mindestens eine Anzeigeeinheit und/oder die mindestens eine Bedieneinheit kann/können in einem mobilen Kommunikationsgerät, insbesondere in einem Mobilfunktelefon, ausgebildet sein.

Des Weiteren ist es möglich, dass die mindestens eine Körper-Lagesensor-Einheit, und/oder die mindestens eine Recheneinheit und/oder die mindestens eine Ausgabeeinheit zur Ausgabe eines akustischen Signals und/oder mindestens eine Ausgabeeinheit zur Ausgabe eines Vibrationssignals und/oder die mindestens eine Anzeigeeinheit und/oder die mindestens eine Bedieneinheit als Teil eines mobilen Kommunikationsgeräts, insbesondere eines Mobilfunktelefons, ausgebildet ist/sind.

Das mobile Kommunikationsgerät kann ein Mobilfunktelefon oder ein Smartphone oder ein PDA oder ein Handheld sein. Bei dem mobilen Kommunikationsgerät kann es sich um ein speziell für die erfindungsgemäße Anwendung ausgebildetes Gerät handeln. Vorzugsweise umfasst das mobile Kommunikationsgerät mindestens eine Funk-Schnittstelle.

Das mobile Kommunikationsgerät kann vorzugsweise Funksignale empfangen und/oder senden. Bei den Funksignalen kann es sich um Bluetooth-Signale und/oder Infrarot-Signale und/oder 3G-Signale und/oder 4G-Signale und/oder LTE-Signale und/oder UMTS-Signale und/oder WLAN-Signale handeln.

Vorzugsweise ist mit Hilfe des mobilen Kommunikationsgerätes eine kabellose Datenübertragung möglich. Die kabellose Datenübertragung kann zwischen der Körper-Lagesensor-Einheit und der Recheneinheit und/oder zwischen der Recheneinheit und der Ausgabeeinheit zur Ausgabe eines akustischen Signals und/oder einer Ausgabeeinheit zur Ausgabe eines Vibrationssignals ausgebildet sein.

Alternativ oder zusätzlich ist die Ausbildung kabelgebundener Datenübertragungswege möglich. Es ist möglich, dass zwischen der Körper-Lagesensor-Einheit und der Recheneinheit und/oder zwischen der Recheneinheit und der Ausgabeeinheit zur Ausgabe eines akustischen Signals und/oder einer Ausgabeeinheit zur Ausgabe eines Vibrationssignals eine Kabelverbindung zur Übertragung von Daten ausgebildet ist.

Bei der mindestens einen Körper-Lagesensor-Einheit kann es sich um einen Lagesensor des mobilen Kommunikationsgeräts handeln. Mit anderen Worten können die Daten und/oder Signale und/oder Informationen mindestens eines Lagesensors eines mobilen Kommunikationsgeräts abgegriffen werden.

Bei der Recheneinheit kann es sich um die Recheneinheit des mobilen Kommunikationsgeräts handeln. Des Weiteren ist es möglich, dass eine vom mobilen Kommunikationsgerät unabhängige Recheneinheit, insbesondere eine externe Recheneinheit, ausgebildet ist.

Bei der Ausgabeeinheit zur Ausgabe eines akustischen Signals kann es sich um einen Kopfhörer handeln, der einem mobilen Kommunikationsgerät zugeordnet ist bzw. Teil eines mobilen Kommunikationsgeräts ist. Der Kopfhörer kann mit dem mobilen Kommunikationsgerät mittels Kabel verbunden sein. Alternativ und/oder zusätzlich ist es möglich, dass zwischen dem Kopfhörer und dem mobilen Kommunikationsgerät eine Funkverbindung besteht oder herstellbar ist.

Die Ausgabeeinheit zur Ausgabe (mindestens) eines stereoakustischen Signals kann ein Knochenleit-Kopfhörer oder ein Funk-Kopfhörer, insbesondere ein Bluetooth-Kopfhörer, oder ein In-Ear-Kopfhörer oder ein Implantat, insbesondere ein Cochlea-Implantat, sein.

Die Ausgabeeinheit zur Ausgabe eines Vibrationssignals kann ein Funk-Vibrationsgeber, insbesondere ein Bluetooth-Vibrationsgeber, oder ein Vibrationsgeber des/eines mobilen Kommunikationsgerätes sein.

Alternativ oder zusätzlich ist es möglich, dass die Ausgabeeinheit zur Ausgabe eines Vibrationssignals als Ausgabeeinheit zur Ausgabe eines Reizstroms ausgebildet ist.

Das System kann außerdem eine Tragevorrichtung für die mindestens eine Körper-Lagesensor-Einheit und/oder die mindestens eine Ausgabeeinheit zur Ausgabe eines akustischen Signals und/oder eine Ausgabeeinheit zur Ausgabe eines Vibrationssignals und/oder für die Recheneinheit und/oder für die Bedieneinheit und/oder für die Anzeigeeinheit umfassen.

Die Tragevorrichtung kann als Tasche mit mindestens einem Gurt ausgebildet sein. Die Tasche kann in Art eines Brustbeutels ausgebildet sein. In der Tasche kann beispielsweise ein mobiles Kommunikationsgerät befindlich sein oder transportiert werden. In der Tasche kann mindestens eine Körper-Lagesensor-Einheit und/oder mindestens eine Ausgabeeinheit zur Ausgabe eines akustischen Signals und/oder mindestens eine Ausgabeeinheit zur Ausgabe eines Vibrationssignals und/oder eine Recheneinheit und/oder eine Bedieneinheit und/oder eine Anzeigeeinheit befindlich sein oder transportiert werden.

Mit Hilfe mindestens eines Brustgurtes und/oder eines Schultergurtes oder kombinierten Brust- und Schultergurten kann die Tasche in ihrer Lage fixiert werden. Vorzugsweise ist die Tasche im Bereich des Solarplexus des Benutzers fixiert.

In einer besonders bevorzugten Ausführungsform der Erfindung ist die Tasche im Bereich des Manubrium sterni des Benutzers fixiert. Es ist vorteilhaft, die Lage des menschlichen Körpers, insbesondere den Neigungsgrad des menschlichen Körpers, in diesem Bereich des Körpers zu erfassen.

Es ist möglich, dass die Tragevorrichtung als Oberkörper-Bekleidungsstück ausgebildet ist. Die Tragevorrichtung kann die Form eines T-Shirts oder eines Langarm-Shirts oder eines Unterhemds aufweisen. Vorzugsweise sitzt eine derartige Tragevorrichtung eng am Körper. Die mindestens eine Körper-Lagesensor-Einheit und/oder die mindestens eine Recheneinheit und/oder die mindestens eine Ausgabeeinheit zur Ausgabe eines akustischen Signals und/oder die mindestens eine Ausgabeeinheit zur Ausgabe eines Vibrationssignals und/oder mindestens eine Bedieneinheit und/oder die mindestens eine Anzeigeeinheit kann/können an dem Oberkörper-Bekleidungsstück befestigt, insbesondere angenäht und/oder angeklebt und/oder angeklettet, sein.

Des Weiteren ist es möglich, dass das Oberkörper-Bekleidungsstück mindestens eine Tasche und/oder ein Fach aufweist, in dem die Körper-Lagesensor-Einheit und/oder die mindestens eine Recheneinheit und/oder die mindestens eine Ausgabeeinheit zur Ausgabe eines akustischen Signals und/oder mindestens eine Ausgabeeinheit zur Ausgabe eines Vibrationssignals und/oder die mindestens eine Bedieneinheit und/oder die mindestens eine Anzeigeeinheit befindlich ist.

Die mindestens eine Körper-Lagesensor-Einheit ist vorzugsweise im Bereich des Solarplexus des Benutzers angeordnet oder fixiert. In einer besonders bevorzugten Ausführungsform der Erfindung ist die mindestens eine Körper-Lagesensor-Einheit im Bereich des Manubrium sterni angeordnet oder fixiert.

Die Ausbildung der Tragevorrichtung als Oberkörper-Bekleidungsstück eignet sich insbesondere dazu, mehrere Lagesensoren auszubilden und an unterschiedlichen Positionen und/oder Körperteilen des Benutzers anzuordnen. Außerdem kann mit Hilfe eines derartig ausgebildeten Oberkörper-Bekleidungsstücks eine Ausgabeeinheit zur Ausgabe eines Vibrationssignals mit mehreren Vibrationsgebern ausgebildet sein. Die Vibrationsgeber können an unterschiedlichen Positionen und/oder an unterschiedlichen Körperteilen des Benutzers angeordnet sein.

Eine weitere Ausbildung der Tragevorrichtung kann strumpfartig oder bandartig ausgebildet sein. Eine derartige Ausbildung der Tragevorrichtung ermöglicht das Anbringen von Lagesensoren, insbesondere von einer Körper-Lagesensor-Einheit, an einer Extremität des Körpers. In einer weiteren Ausführungsform der Erfindung ist es möglich, die Tragevorrichtung handschuhartig auszubilden.

In einer weiteren Ausführungsform der Erfindung ist es möglich, die Tragevorrichtung als Teil eines Schuhs oder schuhartig auszubilden.

Auch die Ausbildung eines Brillen-Adapters ist möglich. Sofern Lagesensoren an einer Brille befestigt sind, kann insbesondere die Kopfhaltung des Benutzers detektiert oder überwacht werden. Auch das Ausbilden einer Ausgabeeinheit zur Ausgabe eines akustischen Signals an einer Brille ist möglich. Eine derartige Ausführungsform der Tragevorrichtung ermöglicht eine Kombination mit bereits vorhandenen Gegenständen bzw. Vorrichtungen des Benutzers.

In einer weiteren Ausführungsform der Erfindung ist es möglich, dass einzelne Elemente des erfindungsgemäßen Systems, insbesondere Lagesensoren, als eine Art Schmuckstück, insbesondere in Form von Ketten oder Broschen, an der Bekleidung des Benutzers befestigt bzw. anbringbar sind. Eine derartige Ausbildung von Lagesensoren und/oder Vibrationsgebern ermöglicht ein unauffälliges Tragen einzelner Komponenten des Systems.

In wiederum einer weiteren Ausführungsform der Erfindung können einzelne Elemente bzw. Bauteil des Systems mit Hilfe von Magneten an der Bekleidung eines Benutzers befestigt oder befestigbar sein. Eine derartige magnetische Befestigung erfolgt vorzugsweise mittels zweier Magneten, wobei zwischen den beiden Magneten die Bekleidung des Benutzers befindlich ist. In diesem Zusammenhang kann die bereits vorhandene Bekleidung des Benutzers als eine Art Tragevorrichtung dienen, wobei die Bekleidung regelmäßig gewechselt werden kann. Eine magnetische Befestigung dient außerdem der individuellen Positionierung einzelner Elemente oder Bauteile des Systems.

In wiederum einer weiteren Ausführungsform der Erfindung kann mindestens ein Sensor, insbesondere mindestens ein Lagesensor, magnetisch an einem transdermalen Implantat befestigt sein.

Das erfindungsgemäße System umfasst ein Audio-Modul. Dieses Audio-Modul ist vorzugsweise zwischen der Recheneinheit und der Ausgabeeinheit zur Ausgabe eines akustischen Signals ausgebildet.

Das Audio-Modul umfasst mindestens eine Tonerzeugungs-Einheit und mindestens eine Audio-Mixer-Einheit. Bei der Tonerzeugungs-Einheit handelt es sich mit anderen Worten um die Audioquelle. Mit Hilfe der Tonerzeugungs-Einheit kann der Grundton des mindestens einen akustischen Signals generiert werden. Dies kann im Sinne eines Synthesizers oder Tongenerators erfolgen. Auch das Ausbilden eines Audio-Players ist möglich. Beispielsweise können Audiodateien gespeichert sein. Mit anderen Worten kann aus mehreren gespeicherten Ton-Sets eines Audio-Players ausgewählt werden. Es ist aber auch möglich, dass ein individueller Ton mittels eines Tongenerators erzeugt wird.

Beispielsweise ist es möglich, dass der Ton im Internet, insbesondere auf einem Server, erzeugt und von der Recheneinheit des Systems und/oder dem Audio-Modul gestreamt, insbesondere abgerufen und empfangen, wird. Dies kann z.B. über einen Internet-Audio-Player erfolgen, wobei die Körper-Lagesensor-Einheit und die Audio-Mixer-Einheit das akustische Signal, vorzugsweise in Echtzeit, erzeugen.

Die Audio-Mixer-Einheit dient zur Ausbildung von Toneffekten sowie der Tonumwandlung. Dies kann sowohl die Tonhöhe als auch die Tonphasen betreffen. Insbesondere wird mit Hilfe der Audio-Mixer-Einheit die Stereo-Lautstärke der einzelnen Kanäle geregelt.

Beispielsweise ist es möglich, dass auch das von der Audio-Mixer-Einheit erzeugte Signal im Internet, insbesondere auf einem Server, erzeugt wird und von der Recheneinheit und/oder dem Audio-Modul gestreamt wird. Verfahrensseitig kann dies z.B. derart erfolgen, dass die Recheneinheit die von der Körper-Lagesensor-Einheit bestimmten Körperlagedaten an einen Server sendet. Das Audio-Mixing und/oder die Tonerzeugung und/oder die Umwandlung der Körperlagedaten kann auf einem Server durchgeführt werden. Das akustische Signal kann von der Recheneinheit des Systems und/oder dem Audio-Modul gestreamt, insbesondere abgerufen und empfangen, werden.

Das System kann außerdem eine Überwachungseinheit, insbesondere eine externe Überwachungseinheit, umfassen. Diese Überwachungseinheit kann als mobiles Kommunikationsgerät ausgebildet sein. Die, vorzugsweise externe, Überwachungseinheit umfasst vorzugsweise eine Bedieneinheit sowie eine Anzeigeeinheit. Die Anzeigeeinheit dient zur visuellen Darstellung der von der Körper-Lagesensor-Einheit ermittelten Daten. Die Bedieneinheit dient vorzugsweise zur Einstellung von Systemparametern. Die Überwachungseinheit ermöglicht es, dass Dritte, wie z. B. Ärzte oder Trainer die Anwendung des erfindungsgemäßen Systems überwachen können und (in Echtzeit) in die Anwendung eingreifen können. Hierbei ist es möglich, dass durch externe Personen wie Ärzte oder Trainer die System-Parameter geändert werden.

Mit Hilfe des erfindungsgemäßen Systems ist es beispielsweise möglich, die Körperhaltung während des Sitzens, insbesondere während eines längeren Sitzens, zu kontrollieren. Es kann somit eine korrekte Sitzhaltung ermöglicht werden. Des Weiteren können einstudierte bzw. festgelegte Bewegungsabläufe, insbesondere im Gang und bei der Körperhaltung, überprüft werden. Der Person kann eine Rückmeldung (Feedback) gegeben werden, sodass die korrekte Ausführung des Bewegungsablaufs und/oder der Bewegungsablauf einzelner Gliedmaßen und/oder einer Übung auch ohne Anwesenheit eines Therapeuten und/oder eines Arztes ermöglicht wird.

In wiederum einer weiteren Ausführungsform der Erfindung kann das System einen Speicher umfassen. Bei dem Speicher kann es sich um einen lokalen Speicher handeln. Dieser lokale Speicher ist vorzugsweise in die Recheneinheit des Systems integriert. Des Weiteren ist es möglich, dass es sich bei dem Speicher um eine externe Datenbank, insbesondere um eine Cloud, handelt. In der Datenbank können die von der Körper-Lagesensor-Einheit erfassten Daten gespeichert und/oder übertragen werden. Die externe Datenbank kann auch als Plattform bezeichnet werden.

Das System kann außerdem eine Kommunikations-Einheit aufweisen. Mit Hilfe der Kommunikations-Einheit können erfasste Daten in die Datenbank, insbesondere zu einer Cloud oder einer Online-Datenbank oder einem Web-Server übertragen werden. Des Weiteren ist die Kommunikations-Einheit vorzugsweise derart ausgestaltet, dass Daten von einer Cloud, einer Online-Datenbank oder einem Webserver abgerufen und in das System, insbesondere in die Recheneinheit, importiert werden können. Dabei handelt es sich um Daten wie Updates der Software, Upgrades der Software, um neue Töne sowie um vordefinierte Presets und/oder Einstellungsmöglichkeiten.

Das erfindungsgemäße Verfahren zur Haltungs- und Bewegungsregulation für Menschen kann auch als Verfahren zur Geh- und Bewegungsregulationshilfe bezeichnet werden. Da die Bewegungsregulation eine Geh- bzw. Gangregulation umfasst und des Weiteren auch bei Stillstand des Nutzers eine Regulation ermöglicht werden soll, nämlich eine Körperhaltungsregulation, wird das Verfahren zur Geh- bzw. Gang- und Bewegungsregulationshilfe im Folgenden als Verfahren zur Körperhaltungs- und Bewegungsregulation für Menschen bezeichnet.

Das erfindungsgemäße Verfahren wird durch Anwendung eines erfindungsgemäßen Systems durchgeführt. Das erfindungsgemäße Verfahren zur Körperhaltungs- und Bewegungsregulation umfasst die folgenden Schritte:
a) Bestimmung der Körperlagedaten des menschlichen Körpers, insbesondere Bestimmung eines Neigungsgrads des menschlichen Körpers,
b) Senden der Körperlagedaten an eine Recheneinheit,
c) Umwandlung der Körperlagedaten in mindestens zwei akustische Signale,
d) Senden der akustischen Signale an eine Ausgabeeinheit zur Ausgabe von zwei stereoakustischen Signalen,
wobei
im Schritt a) die Körperlagedaten in der Sagittalebene und in der Frontalebene des Körpers bestimmt werden und im Schritt c) ein erstes stereoakustisches Signal für die Körperlagedaten in der Frontalebene und ein zweites
stereoakustisches Signal für die Körperlagedaten in der Sagittalebene generiert werden,
wobei im Schritt a) Daten hinsichtlich der Schrittkraft erfasst werden und im Schritt c) in die Berechnung der akustischen Signale einfließen.

Vorzugsweise wird das erfindungsgemäße Verfahren mit dem zuvor beschriebenen erfindungsgemäßen System durchgeführt.

Die Schritte a) bis d) werden vorzugsweise während der Tragedauer des erfindungsgemäßen Systems endlos wiederholt. Der Schritt a) wird vorzugsweise mit Hilfe einer/der Körper-Lagesensor-Einheit durchgeführt.

Im Schritt c) kann ein Vergleich der der Körperlagedaten mit Soll-Lagedaten, insbesondere Soll-Körperlagedaten, durchgeführt werden. Dieser Schritt c) wird vorzugsweise mit Hilfe einer/der Recheneinheit durchgeführt.

Vor dem Schritt a) kann ein Kalibrierungsschritt durchgeführt werden, bei dem die 0°-Lage, insbesondere die 0°-Körperlagedaten, des menschlichen Körpers, insbesondere die 0°-Neigung des menschlichen Körpers, ermittelt wird.

Ein Kalibrierungsschritt ist unter anderem notwendig, um es Personen mit schiefer Körpergrundhaltung ebenfalls zu ermöglichen, das erfindungsgemäße System zu benutzen. Außerdem soll das Gerät schnell am Körper arretiert werden. Da das Gerät jedoch in den überwiegenden Fällen nicht 100 %ig senkrecht angebracht werden kann bzw. wird, ist es notwendig, die 0°-Lage des menschlichen Körpers im Sinne einer Kalibrierung zu bestimmen. Insbesondere kann die 0°-Lage des menschlichen Körpers bzw. die 0°-Neigung des menschlichen Körpers als Mittelwert ermittelt werden. Alternativ ist es möglich, dass das absolute Lot als Neigungsgrad 0 eingestellt wird.

Insbesondere der im Schritt c) vorzugsweise durchgeführte Vergleich der Körperlagedaten mit Soll-Körperlagedaten kann aufgrund der im Kalibrierungsschritt ermittelten 0°-Lage des menschlichen Körpers durchgeführt werden. Die Soll-Körperlagedaten können somit der 0°-Lage des menschlichen Körpers, insbesondere der 0°-Neigung des menschlichen Körpers, entsprechen.

Vor dem Schritt a), insbesondere vor dem Kalibrierungsschritt, kann ein Einstellungsschritt (Setting-mode) durchgeführt werden. Im Einstellungsschritt können beispielsweise ohne vorherige Positionierung eines Lagesensors, insbesondere einer Körper-Lagesensor-Einheit, Voreinstellungen des Systems vorgenommen werden. Derartige Einstellungen sind während des tatsächlichen Bestimmens der Körperlagedaten sowie während der Bewegung nicht mehr oder lediglich sehr schwer möglich.

Im Rahmen des Einstellungsschritts kann zunächst festgelegt werden, bei welchem Neigungsgrad 1 (n1) beginnend ein akustisches Signal ausgegeben werden soll. Des Weiteren ist es möglich, dass im Einstellungsschritt ein Neigungsgrad 2 (n2) definiert wird, bei dem eine Maximallautstärke oder ein Alarm-Ton in Form eines akustischen Signals ausgegeben wird. Des Weiteren ist es möglich, die System-Parameter dahingehend voreinzustellen, ob ein schnell reagierendes und möglicherweise lautes bzw. hartes Signal ausgegeben werden soll oder eine sanft ansteigende akustische Steigerung in einem oder beiden Ohr(en) erzeugt werden soll. Es ist des Weiteren möglich, die Gesamtlautstärke separat einzustellen, die nach Einstellen der System-Parameter in einem Speicher abgelegt wird.

Vorzugsweise werden mindestens zwei Voreinstellungen vorgenommen, um nur den individuellen und relevanten Bewegungs- bzw. Neigungsbereich des menschlichen Körpers in Signale, insbesondere in akustische Signale, umzuwandeln.

Dabei handelt es sich um einen ersten Sensorwert, der beispielsweise einem ersten Neigungswert entspricht. Der erste Sensorwert (n1) definiert den kleinsten Sensorwert, der mindestens überschritten werden muss, um ein akustisches Signal und/oder eine akustische Steigerung zu starten.

Ein/der zweite Sensorwert (n2) kann dem zweiten Neigungswert entsprechen. Der zweite Sensorwert definiert den maximalen Sensorwert, bei dem eine akustische Steigerung maximal erfüllt sein soll. Als akustische Steigerung ist beispielsweise die Tonhöhe bzw. die Lautstärke des akustischen Signals zu verstehen. Als Sensorwerte sind in diesem Zusammenhang also mittels eines Sensors zu detektierende Werte zu verstehen.

Die beiden Sensorwerte (n1, n2) können jeweils für eine Bewegung nach links und nach rechts bestimmt oder festgelegt werden. Die beiden Sensorwerte (n1, n2) können jeweils für eine Bewegung nach vorne und nach hinten bestimmt oder festgelegt werde.

Dabei ist es möglich, dass die Sensorwerte (n1, n2) von zwei Seiten, d. h. für links und rechts oder für vorne und hinten symmetrisch zu einer mittleren Köperbezugsachse bestimmt oder festgelegt werden. D. h., dass die beiden Sensorwerte (n1, n2) lediglich für eine Seite (z.B. links) bestimmt werden und anschließend die beiden Sensorwerte (n1, n2) für die zweite Seite (z.B. rechts) symmetrisch hierzu festgelegt werden.

In wiederum einer weiteren Ausführungsform der Erfindung ist es möglich, zunächst die Sensorwerte (n1, n2) für eine erste Seite und anschließend für die zweite Seite zu bestimmen. Es kann somit eine bezüglich der jeweiligen Seiten voneinander unabhängige Bestimmung der Sensorwerte (n1, n2) stattfinden.

In einer ersten Ausführungsform des erfindungsgemäßen Verfahrens können die Voreinstellungen im Einstellungsschritt mittels einer Bedieneinheit vorgenommen werden. Es ist möglich, dass sich der Benutzer an möglichen Einstellwerten bzw. an einer Auswahl von vorgegebenen System-Parametern orientiert und somit den ersten Sensorwert (n1) und den zweiten Sensorwert (n2) definiert.

In einer weiteren Ausführungsform des erfindungsgemäßen Verfahrens ist es möglich, dass der Einstellungsschritt nicht im Sinne einer einzutippenden Voreinstellung bzw. einer aus einer Menge von Voreinstellungen auszuwählenden Voreinstellung erfolgt. Der Einstellungsschritt ist in dieser Ausführungsform des erfindungsgemäßen Verfahrens ohne Sicht bzw. ohne Blick auf eine Bedieneinheit möglich. In dieser Ausführungsform ist die Voreinstellung bzw. der Einstellungsschritt mittels Tippen auf eine Bedieneinheit durchführbar. Der Benutzer orientiert sich dabei an einer Echtzeit-Körperneigung. Während diese Echtzeit-Körperneigung vom Benutzer aktiv durchgeführt wird, kann durch Bestätigung auf der Bedieneinheit, d. h. durch blindes Tippen, jeweils ein erster Sensorwert und ein zweiter Sensorwert eingestellt werden. Die Bedieneinheit dient in diesem Fall als eine Art Response-Button, d. h. als Rückmeldungs-Knopf.

Im Schritt a) können die Körperlagedaten in der Sagittalebene und in der Frontalebene des Körpers bestimmt werden. Die Sagittalebene bezeichnet dabei eine sich vom Kopf zum Becken und vom Rücken zum Bauch erstreckende Ebene. Als Frontalebene wird die bei einer Vorderansicht des Menschen sichtbare Bewegungsebene bezeichnet. Im Zusammenhang mit der Sagittalebene werden demnach die Körperlagedaten im Sinne einer Erstreckung von vorne nach hinten oder umgekehrt erfasst. Im Zusammenhang mit der Frontalebene werden die Körperlagedaten in einer Erstreckung von links nach rechts bzw. umgekehrt erfasst.

Im Schritt c) werden ein erstes akustisches Signal für die Körperlagedaten in der Frontalebene und ein zweites akustisches Signal für die Körperlagedaten in der Sagittalebene generiert. Mit anderen Worten wird bei einer Neigung des Körpers nach links oder nach rechts ein erster Stereo-Ton generiert werden. Bei einer Körperneigung nach vorne oder nach hinten wird ein zweiter Stereo-Ton generiert.

Mit anderen Worten kann durch einen zum Neigungsgrad innerhalb eines definierten Bereichs ansteigender Ton im jeweils zugehörigen Ohr, d. h. im linken oder im rechten Ohr, und durch einen sich zusätzlich sich ändernden Stereo-Toncharakter (vorne, hinten) dem Benutzer akustisch in Echtzeit intuitiv vermittelt werden, in welche Richtung und wie stark die Körperneigung gerade ausgebildet ist. In Abhängigkeit der Voreinstellungen kann der Tonanstieg langsam oder schnell erfolgen.

Es ist möglich, einen sogenannten Basiston zu erzeugen. Ein derartiger Basiston wird über die Ausgabeeinheit zur Ausgabe eines akustischen Signals auch dann ausgegeben, wenn keine Abweichung von der 0°-Lage des menschlichen Körpers, insbesondere keine Abweichung von der 0°-Neigung des menschlichen Körpers, vorliegt. Der Basiston kann äußerst leise sein. Ein Basiston dient dazu, die erzeugten Tonunterschiede sensitiver wahrzunehmen. Außerdem dient der Basiston dazu, eine permanente System-Kontrolle zu erzielen. Sofern kein Basiston ausgegeben wird, kann der Benutzer darauf schließen, dass das System nicht funktioniert bzw. eine Störung vorliegt.

Vorzugsweise werden die Körperlagedaten gespeichert. Außerdem ist es möglich, dass die Körperlagedaten an eine Datenbank gesendet werden.

Die Körperlagedaten können in der erfindungsgemäßen Recheneinheit gespeichert werden. Außerdem ist es möglich, dass ein mobiles Kommunikationsgerät einen Speicher zur Ablage der Körperlagedaten aufweist. Auch das Ausbilden eines externen Speichers ist möglich.

Sofern die Körperlagedaten an eine Datenbank gesendet werden, handelt es sich vorzugsweise um eine externe Datenbank. Bei der Datenbank kann es sich um eine Cloud handeln.

Des Weiteren ist es möglich automatisch erfasste Daten zu speichern oder an eine Datenbank zu senden. Bei den automatisch erfassten Daten kann es sich beispielsweise um die Uhrzeit und/oder das Datum und/oder die Wetterbedingungen und/oder die Temperatur und/oder ein GPS-Signal und/oder die Bewegungs- und Erschütterungswerte und/oder die zurückgelegte Wegstrecke und/oder die Verwendungsdauer des Systems und/oder die Bewegungsgeschwindigkeit und/oder Informationen über die Bodenbeschaffenheit und/oder die verwendeten Systemeinstellungen, insbesondere die festgelegten Sensorschwellenwerte, handeln. Somit ist es möglich, die gespeicherten Körperlagedaten in Zusammenhang zu äußeren Bedingungen zu setzen.

Außerdem ist es vorzugsweise möglich, manuell erstellte Notizen des Benutzers zu speichern oder an eine Datenbank zu senden. Bei manuell erstellten Notizen kann es sich beispielsweise um das Krankheitsbild des Benutzers und/oder um die aktuelle Medikamentation des Benutzers und/oder den allgemeinen Gesundheitszustand des Benutzers handeln. Dies ermöglicht das Durchführen eines Analysemodus. Es können Therapieverläufe aufgezeichnet und optimiert werden.

Durch einen dem Neigungsgrad des Körpers zugeordneten Ton im jeweiligen Ohr eines Benutzers, wird der Benutzer, insbesondere des erfindungsgemäßen Systems, akustisch darüber informiert, in welche Richtung der Körper geneigt ist und/oder wie stark der Körper geneigt ist.

Mit anderen Worten kann ein der Abweichung des Körpers von der 0°-Lage des menschlichen Körpers zugeordnetes akustisches Signal im jeweiligen Ohr eines Benutzers, den Benutzer darüber informieren, in welche Richtung der Körper von der 0°-Lage des menschlichen Körpers abweicht und/oder wie stark die Körperlage von der 0°-Lage des menschlichen Körpers abweicht.

Das akustische Signal kann auch ein stummes Signal sein. Sofern kein Ton, insbesondere kein stereoakustisches Signal, ausgegeben wird, ist die Lage/Neigung des Körpers nicht zu korrigieren. Gleiches gilt für die Ausgabe eines Vibrationssignals. Sofern die Lage/Neigung des Körpers nicht korrigiert werden muss, ist es möglich, kein Vibrationssignal auszugeben.

Eine erste Methode zur Umwandlung der Körperlagedaten in ein akustisches Signal sieht einen Mono-Anstieg vor. Sofern an ein Ohr ein geändertes akustisches Signal, insbesondere ein sich hinsichtlich der Lautstärke oder der Modulation oder hinsichtlich der Effekte sich änderndes akustisches Signal, gesendet wird, ist die Lage des Körpers zu korrigieren. Sofern kein akustisches Signal von der Ausgabeeinheit ausgegeben wird oder lediglich der Basiston von der Ausgabeeinheit zur Ausgabe eines akustischen Signals ausgegeben wird, ist die Körperlage bzw. die Neigung des Körpers nicht zu korrigieren.

Eine weitere Ausführungsform des Schrittes c), d. h. der Umwandlung der Körperlagedaten in mindestens ein akustisches Signal, sieht vor, dass ein Stereo-Wechsel durchgeführt wird. Demnach wird bei Einnahme einer 0°-Lage des menschlichen Körpers, insbesondere einer 0°-Neigung des menschlichen Körpers, an beide Ohren des Benutzers ein mittiges, gleichwertiges Stereosignal ausgegeben. Mit anderen Worten wird ein Stereosignal zu gleichen Anteilen an das linke und an das rechte Ohr des Benutzers ausgegeben. Bei einer zu korrigierenden Körperlage, insbesondere bei einer zu korrigierenden Neigung des menschlichen Körpers, wird auf einem Ohr das akustische Signal gesteigert und gleichwertig auf dem anderen Ohr reduziert.

Das erfindungsgemäße Verfahren sieht insbesondere vor, dass auch bei einer Abweichung der Körperlage nach vorne oder nach hinten ein, insbesondere zweites, akustisches Signal von der Ausgabeeinheit ausgegeben wird. Das zweite akustische Signal kann in Abhängigkeit von vordefinierten Einstellungen und/oder in Abhängigkeit des tatsächlichen Neigungsgrades das erste akustische Signal zunehmend ersetzen.

Abhängig von dem persönlichen Empfinden des Benutzers und dem Therapieziel, kann das akustische Signal eine Art von Druck-Widerstand oder eine Art von Sogwirkung beim Benutzer erzeugen.

Bei einem Verfahren nach Art eines Druck-Widerstands steigt die Lautstärke des Tons in dem Ohr des Benutzers an, zu welcher Seite sich der Körper des Benutzers neigt.

Mit anderen Worten nimmt bei einer Neigung des Körpers zu einer ersten Seite die Lautstärke des Tons im Ohr dieser ersten Seite zu. Der Ton signalisiert also die Ist-Situation der Körperlage bzw. des Neigungszustandes des Körpers des Benutzers.

Nach einer Eingewöhnungszeit empfindet der Benutzer die akustische Signalausgabe als ansteigenden Widerstand und steigende Warnung.

Bei einem Verfahren nach Art einer Sogwirkung wird das akustische Signal auf dem zur Neigungsrichtung entgegengesetzten Ohr erzeugt. Nach einer Eingewöhnungszeit empfindet der Benutzer eine derartige akustische Signalausgabe als weiche angenehme Korrektur und weniger als Warnung.

Das Verfahren nach Art eines Druck-Widerstands oder das Verfahren nach Art einer Sogwirkung kann alternativ oder zusätzlich mit Vibrationssignalen durchgeführt werden.

In einer weiteren Ausführungsform der Erfindung ist es möglich, dass im Schritt a) die durchschnittliche Bewegungsrichtung des Körpers und eine Abweichung von der durchschnittlichen Bewegungsrichtung bestimmt werden. Es ist möglich, dass ein Benutzer ein schiefes Gangbild aufweist, ohne dass die Körperlage von der 0°-Körperlage abweicht oder der Körper in eine bestimmte Richtung geneigt ist. Es ist demnach möglich, dass ein Benutzer trotz geradem Oberkörper, mit anderen Worten trotz einer geraden Körperhaltung, beispielsweise durch schwankende Schritte, von der durchschnittlichen Bewegungsrichtung abweicht. Demnach ist es möglich, dass Benutzern Abweichungen von der durchschnittlichen Bewegungsrichtung durch ein akustisches Signal mitgeteilt werden können. Hierzu muss zunächst die durchschnittliche Bewegungsrichtung des Benutzers erkannt oder detektiert werden.

Auch bei der Einbeziehung von Abweichungen von der durchschnittlichen Bewegungsrichtung bei der Umwandlung (Schritt c)) der Körperlagedaten in mindestens ein akustisches Signal kann ein dem Schritt c) vorangehender Einstellungsschritt (Setting-mode) erfolgen.

Vor dem Kalibrierungsschritt, kann demnach ein Einstellungsschritt (Setting-mode) durchgeführt werden.

Im Rahmen des Einstellungsschritts kann zunächst festgelegt werden, bei welchem Abweichungsgrad (a1) von der durchschnittlichen Bewegungsrichtung beginnend ein akustisches Signal ausgegeben werden soll. Des Weiteren ist es möglich, dass im Einstellungsschritt ein Abweichungsgrad 2 (a2) definiert wird, bei dem eine Maximallautstärke oder ein Alarm-Ton in Form eines akustischen Signals ausgegeben wird. Des Weiteren ist es möglich, die System-Parameter dahingehend voreinzustellen, ob ein schnell reagierendes und möglicherweise lautes bzw. hartes Signal ausgegeben werden soll oder eine sanft ansteigende akustische Steigerung in einem oder beiden Ohren erzeugt werden soll.

Auch das Durchführen des Einstellungsschritts, d. h. entweder die Auswahl von Voreinstellungen aus einer Vielzahl von zur Verfügung stehenden Voreinstellungen oder das Einstellen anhand einer Echtzeit-Bewegung kann im Zusammenhang mit dem Abweichungsgrad derart erfolgen, wie dies bereits im Zusammenhang mit dem Neigungsgrad beschrieben ist.

Die Abweichung von der durchschnittlichen Bewegungsrichtung kann alternativ und/oder zusätzlich zu einem Neigungswert akustisch wiedergegeben werden. Die Option hinsichtlich der Einbeziehung der Abweichung von der durchschnittlichen Bewegungsrichtung kann zu- und abschaltbar ausgebildet sein.

Erfindungsgemäß ist es vorgesehen, dass bei der Bestimmung der Körperlagedaten im Schritt a) Daten hinsichtlich der Schrittkraft, die auch als Schrittstärke oder Auftrittskraft bezeichnet werden kann, erfasst werden. Als Schrittkraft bzw. Schrittstärke bzw. Auftrittskraft ist die Kraft zu verstehen, mit welcher der Fuß beim Gehen auf der Oberfläche auftrifft. Die Schrittkraft wird vom dem erfindungsgemäßen System beispielsweise als Körpererschütterung detektiert. Erfindungsgemäß umfasst die Körper-Lagesensor-Einheit zur Detektion dieser Körpererschütterung mindestens einen Erschütterungssensor.

Die Daten hinsichtlich der Schrittkraft werden im Schritt c), d. h. bei der Umwandlung der Körperlagedaten in mindestens ein akustisches Signal beachtet. Mit anderen Worten fließen die Daten hinsichtlich der Schrittkraft in die Berechnung des akustischen Signals ein.

Es ist möglich, diese Daten der Schrittkraft bei der Erzeugung des akustischen Signals im Vergleich zu den zu Köperneigungs-Daten zugehörigen akustischen Signalen, verstärkt auszugeben. Dies dient insbesondere dazu, verstärkt akustische Signale hinsichtlich des Gangbilds auszugeben. Der Benutzer kann dadurch Informationen über sein Gangbild erhalten.

Des Weiteren ist es möglich, die Daten der Schrittkraft bei der Erzeugung des akustischen Signals nicht mit einzubeziehen oder aktiv herauszufiltern. Für Benutzer, die lediglich Informationen über eine Körperschwankung erhalten wollen, kann das zusätzliche Ausgeben eines akustischen Signals im Zusammenhang mit der Schrittkraft störend wirken. Der Benutzer kann sich bei einem Herausfiltern des Signals der Schrittkraft besser auf die Körperschwankung und auf das Ausgleichen der Körperschwankung konzentrieren.

In einer weiteren Ausführungsform der Erfindung können im Schritt a) Daten hinsichtlich der Schrittlänge und/oder der zeitlichen Dauer des Bodenkontakts der beiden Füße und/oder der Abstoßhöhe der Füße erfasst werden. Diese Daten können im Schritt c), d. h. bei der Umwandlung der Körperlagedaten in mindestens ein akustisches Signal beachtet werden. Mit anderen Worten können die genannten Daten in die Berechnung des akustischen Signals einfließen.

Des Weiteren ist es möglich, dass die in Schritt a) bestimmten Körperlagedaten, insbesondere die bestimmten Neigungsgraddaten, in ein visuelles Abbild umgewandelt werden und an eine Anzeigeeinheit gesendet und/oder in einer Datenbank gespeichert werden.

Die Aufgabe wird ferner gelöst durch ein computerlesbares Speichermedium, welches Instruktionen enthält, die mindestens einen Prozessor dazu veranlassen, ein erfindungsgemäßes Verfahren nach einer der vorhergehenden Ausführungsformen zu implementieren, wenn die Instruktionen durch den Prozessor ausgeführt werden.

Es ergeben sich ähnliche oder identische Vorteile, wie diese bereits mit dem vorstehend beschriebenen Verfahren beschrieben wurden.

Zusätzlich oder alternativ wird auf die schematischen Darstellungen verwiesen, die das erfindungsgemäße System zur Geh- und Bewegungsregulationshilfe, insbesondere das erfindungsgemäße System zur Körperhaltungs- und Bewegungsregulation, und/oder das erfindungsgemäße Verfahren zur Geh- und Bewegungsregulationshilfe, insbesondere das erfindungsgemäße Verfahren zur Körperhaltungs- und Bewegungsregulation für Menschen, (näher) beschreiben.

Dabei zeigen:
- Fig. 1a und 1b: eine Prinzipdarstellung des erfindungsgemäßen Systems zur Körperhaltungs- und Bewegungsregulation;
- Fig. 2a bis 2c: verschiedene Darstellungen von Tragevorrichtungen des erfindungsgemäßen Systems;
- Fig. 3a bis 3d: beispielhafte Darstellungen auf der Anzeigeeinheit;
- Fig. 4a bis 4e: verschiedene Darstellungen auf einer Bedieneinheit in Abhängigkeit des jeweils ausgeführten Verfahrensschritts;
- Fig. 5a bis 5e: Prinzipdarstellungen hinsichtlich der Umwandlung der Körperlagedaten in mindestens ein akustisches Signal und/oder ein Vibrationssignal;
- Fig. 6: Übersicht möglicher Elemente bzw. Bauteile des erfindungsgemäßen Systems; und
- Fig. 7a und 7b: Darstellung weiterer Anbindungsmöglichkeiten an erfindungsgemäßes System zur Körperhaltungs- und Bewegungsregulation.

Im Folgenden werden für gleiche oder gleichwirkende Teile dieselben Bezugsziffern verwendet.

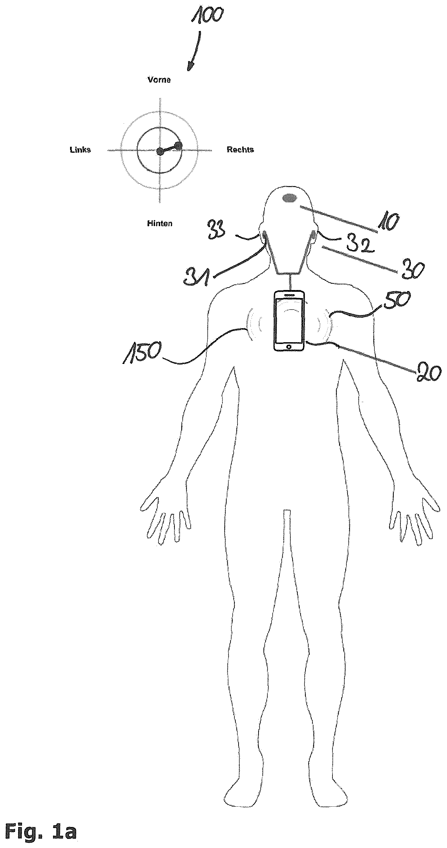

Wie in Fig. 1a dargestellt ist, werden Neigungsdaten von Lagesensoren eines am Körper 10 befestigten mobilen Kommunikationsgerätes, insbesondere eines Smartphones 20, in akustische, insbesondere stereoakustische Signale 30, umgewandelt. Im Smartphone 20 sind alle notwendigen Elemente des erfindungsgemäßen Systems ausgebildet. Bei den notwendigen Elementen handelt es sich um mindestens einen Lagesensor, insbesondere um eine Körper-Lagesensor-Einheit. Auch eine Recheneinheit ist im Smartphone 20 integriert. Des Weiteren ist eine Ausgabeeinheit zur Ausgabe eines akustischen Signals, in diesem Falle eines stereoakustischen Signals 30, ausgebildet.

Die Ausgabeeinheit ist in diesem Fall ein Kopfhörer 31. Am Körper 10 direkt oder indirekt anliegende Lagesensoren bzw. eine Körper-Lagesensor-Einheit kann Daten hinsichtlich der Position und/oder Neigung und/oder Rotation und/oder Haltung sowie Positions- und/oder Neigungs- und/oder Rotations- und/oder Haltungsänderungen an die Recheneinheit des Smartphones 20 senden. Diese Daten werden in Echtzeit in mindestens ein akustisches Signal und/oder ein Vibrationssignal umgewandelt. Im vorliegenden Fall werden die Daten in stereoakustische Signale umgewandelt. Die stereoakustischen Signale 30 werden im linken Ohr 32 und/oder im rechten Ohr 33 ausgegeben bzw. dort wahrgenommen. Der Benutzer kann auf das stereoakustische Signal reagieren und die Körperlage ändern. Die Lagesensoren sind im vorliegenden Fall in das Smartphone 20 integriert. Außerdem kann das Smartphone 20 eine Ausgabeeinheit 150 für Vibrationssignale umfassen. Dies ist durch die ausgesendeten Vibrationssignale 50 schematisch angedeutet.

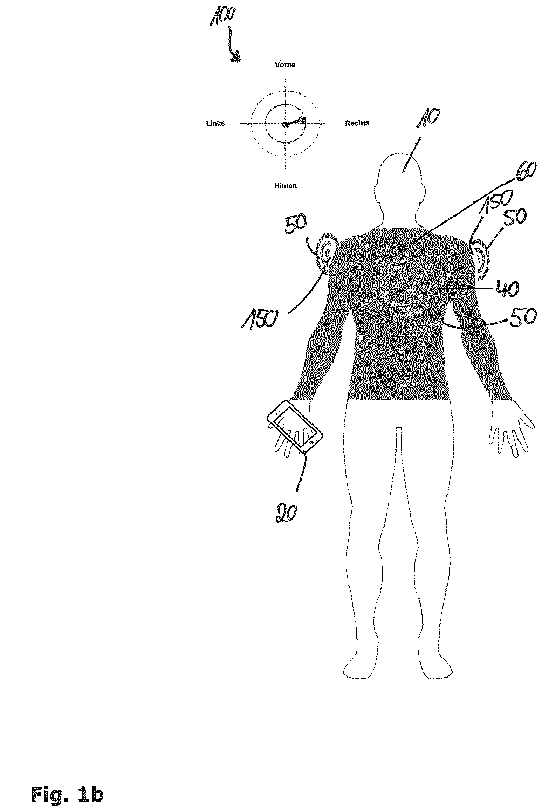

Wie in Fig. 1b dargestellt ist, kann ein Lagesensor 40, beispielsweise über eine Funkverbindung mit einem mobilen Kommunikationsgerät, insbesondere einem Endgerät 20 mit Internetzugang, verbunden sein. Die Lagedaten/Neigungsdaten, insbesondere die Körperlagedaten, werden im dargestellten Beispiel in Vibrationssignale 50 umgewandelt. Auf dem Bildschirm das mobilen Kommunikationsgeräts, insbesondere des Smartphones 20, kann eine bildliche Darstellung 100 anzeigen, welche Lage der Körper 10 des Benutzers einnimmt bzw. inwiefern der Körper 10 des Benutzers geneigt ist.

Da mehrere Vibrationsgeber an der Tragevorrichtung 60 befestigt sind, können an verschiedenen Punkten des Körpers Vibrationssignale abgegeben werden.

In Fig. 1a werden die stereoakustischen Signale über Kopfhörer 31, In-Ears oder Knochenleit-Kopfhörern, per Kabel oder Bluetooth direkt am Ohr, insbesondere an den beiden Ohren 32 und 33 wahrgenommen. Vibrationssignale können in Fig. 1 direkt über das Smartphone 20 ausgelöst und auf den Körper 10 übertragen werden. In Fig. 1b sind hingegen alternative externe Bluetooth-Vibrationsgeber 150 in unmittelbarer Nähe zum Körper 10 angeordnet.

Das Ziel dieses erfindungsgemäßen Systems/Verfahrens ist es, Patienten mit Gleichgewichtsstörungen, bei Rehabilitationsphasen nach Unfällen/Operationen oder bei zu korrigierenden Fehlhaltungen ein zusätzliches, einfach verständliches Feedback zur eigenen Körperhaltung bzw. Schräglage bzw. Körperneigung zu geben und zusätzlich durch den Lerneffekt des "neuen Sinnes" ein sicheres Gefühl und Stabilitätsempfinden beim Benutzer zu bewirken. Außerdem können dem Benutzer oder Ärzten oder Forschern oder Trainern oder Physiotherapeuten die Bewegungsdaten zur Verfügung gestellt werden.

In Fig. 1b ist eine Tragevorrichtung 60 in Form eines Langarm-Shirts ausgebildet. Lagesensoren 40 und Bluetooth-Vibrationsgeber 150 sind in die Tragevorrichtung 60 z. B. durch Verkleben, Vernähen oder Ankletten integriert.

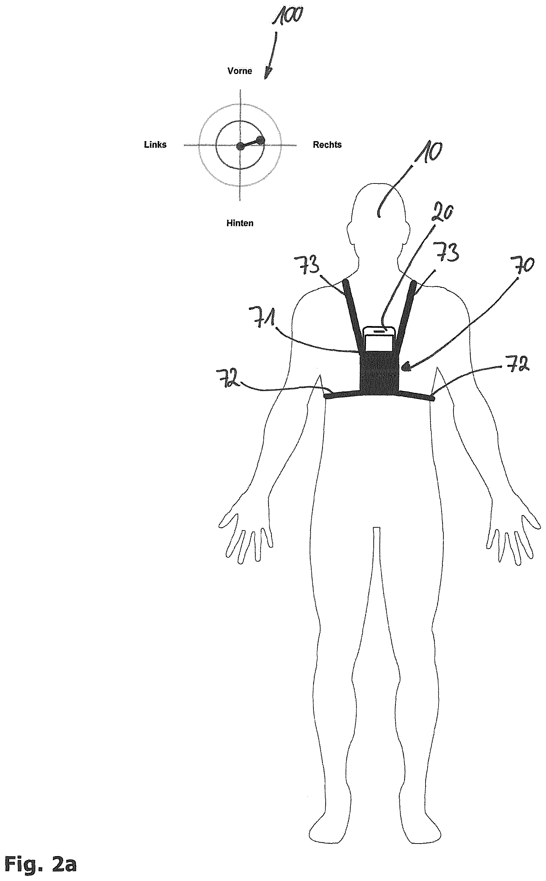

Zur Position und Nutzung am Körper ist anzumerken, dass ein Smartphone 20 auch wahlweise auf der Körpervorderseite oder am Rücken durch eine Tragevorrichtung 70 befestigt werden kann, wie dies in Fig. 2a dargestellt ist. In dieser Tragevorrichtung 70 befindet sich optional eine Akkuerweiterung/ein Zusatz-Akku für Smartphones. Die Tragevorrichtung 70 ist als Tasche 71 mit einem Bauchgurt 72 und Schultergurten 73 ausgebildet.

In Zusammenhang mit den Sensoren, insbesondere den Lagesensoren, insbesondere der Körper-Lagesensor-Einheit ist anzumerken, dass die Neigungssensordaten "vorne", "hinten", "links" und "rechts", die von dem Smartphone 20 abgegriffen werden, in Echtzeit über eine Applikation (App) in Gradzahlen abgegriffen und visuell dargestellt werden. Die Darstellung 100 zeigt, dass der Körper leicht nach vorne rechts geneigt ist. Die Applikation steht vorzugsweise im Zusammenhang mit einem in der Recheneinheit des erfindungsgemäßen Systems abgelegten Rechenprogramm.

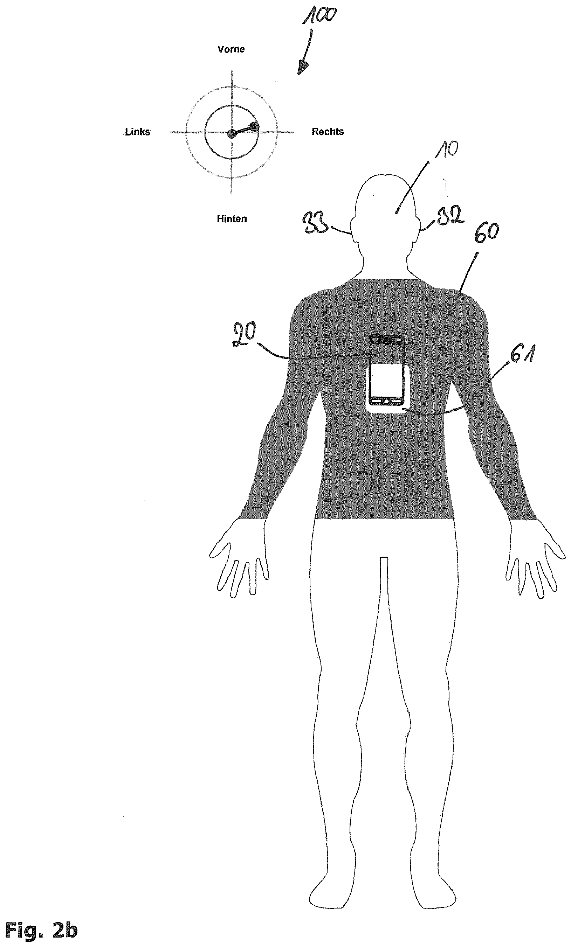

In Fig. 2b ist wiederum eine Tragevorrichtung 60 in Form eines Langarm-Shirts dargestellt. Diese Tragevorrichtung 60 weist eine Tasche 61 auf. Diese Tasche 61 kann ein Smartphone 20 aufnehmen. In dem Smartphone 20 sind wiederum alle notwendigen Bauteile bzw. Elemente des erfindungsgemäßen Systems integriert. Dabei handelt es sich um die Körper-Lagesensor-Einheit, die Recheneinheit, sowie die Ausgabeeinheit zur Ausgabe eines akustischen Signals. Im dargestellten Beispiel sind Kopfhörer nicht mittels Kabel, sondern mittels Funkverbindung an das Smartphone 20 angeschlossen.

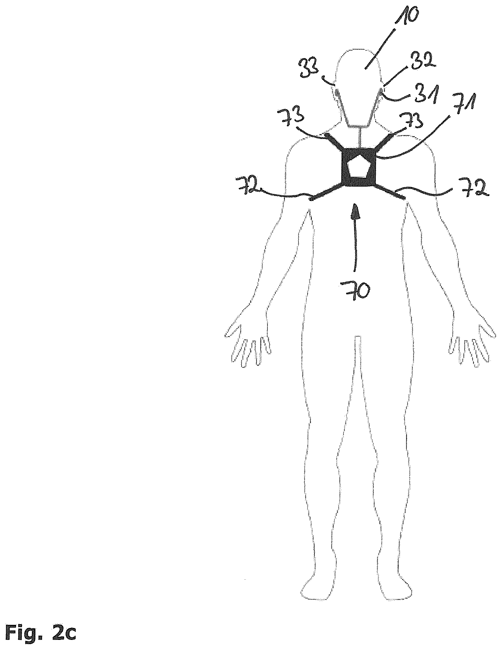

In Fig. 2c ist eine weitere Tragevorrichtung 70 dargestellt. Diese umfasst wiederum eine Tasche 71. In dieser Tasche 71 kann ein mobiles Kommunikationsgerät befindlich sein. Wie in Fig. 2c verdeutlicht ist, ist die Tasche 71 derart am Körper 10 angeordnet, dass diese im Bereich des Manubrium sterni angeordnet ist. Somit können die Körperlagedaten des Körpers 10 im Bereich des Manubrium sterni detektiert werden. Die Detektion erfolgt durch ein in der Tasche 71 befindliches Kommunikationsgerät, das eine Körper-Lagesensor-Einheit aufweist.

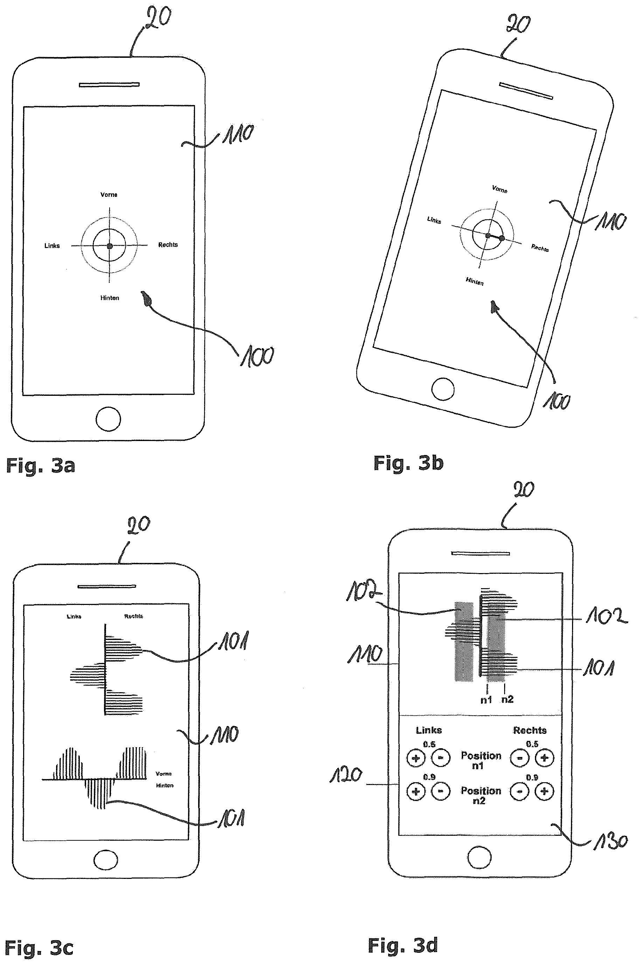

In den Fig. 3a bis 3d sind beispielhafte Darstellungen 100 auf dem mobilen Kommunikationsgerät 20 dargestellt. Die in den Fig. 3a und 3b abgebildeten 2-Achsendarstellungen ähneln einer Wasserwaagen-Ansicht von oben (Dosenlibelle). In diesen Darstellungen 100 werden beim Gehen die Bewegungen auf beiden Achsen, d. h. die Bewegungen in der Sagittalebene sowie in der Frontalebene in einer Ansicht 100 dargestellt. Dadurch wird eine bessere Einschätzung eines komplexen Bewegungsablaufes ermöglicht. In Fig. 3a befindet sich der Körper in einer 0°-Lage. Es ist somit keine Anpassung der Körperlage vorzunehmen.

In Fig. 3b wird hingegen eine Neigung des Smartphones 20 angedeutet. Die im Smartphone 20 integrierten Lagesensoren detektieren somit eine Abweichung von der 0°-Körperlage. In der Darstellung 100 wird folglich dargestellt, dass eine Neigung nach rechts vorliegt. In den Fig. 3a und 3b wird der Bildschirm des Smartphones als Anzeigeeinheit 110 des erfindungsgemäßen Systems genutzt.

Die Anzeigeeinheit 110 dient zur visuellen Darstellung der von der Körper-Lagesensor-Einheit ermittelten Daten.

In Fig. 3c wird der Bildschirm des Smartphones 20 ebenfalls als Anzeigeeinheit 110 genutzt. Die Bewegungen werden in diesem Fall als Bewegungsskala bzw. als Graphen 101 dargestellt. Mit Hilfe einer derartigen Bewegungsskala bzw. derartiger Graphen können ältere gespeicherte Bewegungsskalen mit der aktuellen Bewegungsskala verglichen werden. Außerdem ist ein Abgleich der Bewegungsskala bzw. der Graphen 101 mit zuvor eingestellten Bewegungsbereichen möglich.

In Fig. 3d wird wiederum der Bildschirm eines Smartphones 20 dargestellt.
Der Bildschirm dient sowohl als Anzeigeeinheit 110 als auch als Bedieneinheit 120. In der Anzeigeeinheit 110 wird ein Graph 101, wie dieser in Fig. 3c gezeigt ist, dargestellt. Des Weiteren werden in der Anzeigeeinheit 110 aktive Bereiche 102 dargestellt. Als aktive Bereiche 102 sind die Neigungsbereiche bzw. Abweichungsbereiche von der 0°-Körperlage definiert, in denen vorzugsweise akustische Signale ausgegeben werden. Die aktiven Bereiche 102 werden vorzugsweise in einem Einstellungsschritt definiert.

Der Einstellungsschritt (Setting-mode) kann ohne das Anbringen von Lagesensoren bzw. ohne Anbringen einer Körper-Lagesensor-Einheit durchgeführt werden. Eine erste Möglichkeit zur Festlegung der aktiven Bereiche erfolgt mit Hilfe der Bedieneinheit 120. Dabei werden erste Sensorwerte bzw. erste Neigungswerte (n1) definiert. Diese sind die kleinsten festgestellten Abweichungen von der 0°-Körperlage, die mindestens überschritten werden müssen, um das Ausgeben eines akustischen Signals auszulösen.

Des Weiteren sind zweite Sensorwerte, insbesondere zweite Neigungswerte (n2) festzulegen. Diese Neigungswerte n2 definieren den maximalen Abweichungsgrad von der 0°-Körperlage, bei der das akustische Signal maximal ausgegeben wird. Dies kann bezüglich der Tonhöhe bzw. Tondauer als maximal bezeichnet werden.

Der Einstellungsschritt im Zusammenhang mit der Bedieneinheit 120 kann derart durchgeführt werden, dass sich der Benutzer an den möglichen Einstellwerten und deren visualisierten Bereichen in der Bedieneinheit 120 orientiert und durch Werteänderungen, nämlich durch Betätigung der Plus-Tasten bzw. Minus-Tasten die Schwellen-Sensorwerte n1 und n2 festlegt. Der zusätzlich angezeigte Graph 101 visualisiert die aufgetretenen Bewegungsdaten und dient zum Abgleich der Feineinstellungen.

Eine weitere Ausführungsform eines Einstellungsschritts sieht eine Festlegung der Minimum- und Maximum-Werte bzw. eine Festlegung der ersten und zweiten Sensorwerte (n1, n2) lediglich durch Tippen auf die Bedieneinheit 120 vor. Zum Durchführen des Einstellungsschritts gemäß zweiter Ausführungsform ist es zunächst notwendig, in der Bedieneinheit 120 den Einstellungsschritt bzw. den Setting-mode zu starten. Anschließend führt der Benutzer eine Echtzeit-Körperneigung durch und legt aufgrund dessen die Minimum- und Maximum-Werte, d. h. die ersten und zweiten Sensorwerte fest. Dies kann beispielsweise wie folgt durchgeführt werden:
Zunächst erfolgt eine leichte Neigung auf eine erste Seite (links oder rechts). Durch Bestätigung der leichten Körperneigung kann der erste Sensorwert (≙ Neigungswert 1 = n1) festgelegt werden. Dabei muss der Benutzer keine spezielle Taste bzw. kein spezielles Feld der Bedieneinheit 120 treffen, sondern kann lediglich durch Tippen auf das Bedienfeld 120 diesen Neigungswert bestätigen. In diesem Zusammenhang dient die Bedieneinheit 120 als eine Art Rückmeldungs-Taster.

Anschließend wird eine stärkere Körperneigung in die ausgewählte Richtung (links oder rechts) durchgeführt. Durch Tippen auf das Bedienfeld 120 wird wiederum der zweite Sensorwert (≙ Neigungswert 2 = n2) bestätigt.

In der Voreinstellung können anschließend die festgelegten Sensorwerte 1 und 2 für die andere Körperseite (links oder rechts) gespiegelt eingestellt werden. Auf der verbleibenden Seite werden also die gleichen Werte n1 und n2 wie für die zuvor festgelegten Werte n1 und n2 eingestellt. Es ist auch möglich, dass die Sensorwerte 1 und 2 für die weitere Körperseite separat von der ersten Körperseite eingestellt werden.

In einer Ausführungsform der Erfindung ist es möglich, dass der Einstellungsschritt (Setting-mode) durch eine Sprachansage erklärt wird und somit die einzelnen Schritte, wie z. B. das zunächst leichte Neigen und das anschließend stärkere Neigen, Schritt für Schritt anhand der Sprachansage ausgeführt werden können. Der Einstellungsschritt gemäß zweiter Ausführungsform kann auch als Quick-Tap-Configuration bezeichnet werden. Auch ein Therapeut und/oder ein Arzt und/oder ein Forscher kann diese zweite Ausführungsform des Einstellungsschrittes nutzen. Dies kann beispielsweise durch eine/die Überwachungs-Einheit erfolgen.

Wie in Fig. 3d gezeigt wird, können die Anzeigeeinheit 110 sowie die Bedieneinheit 120 in einem Ein- und Ausgabemodul 130 in kombinierter Art und Weise vorliegen.

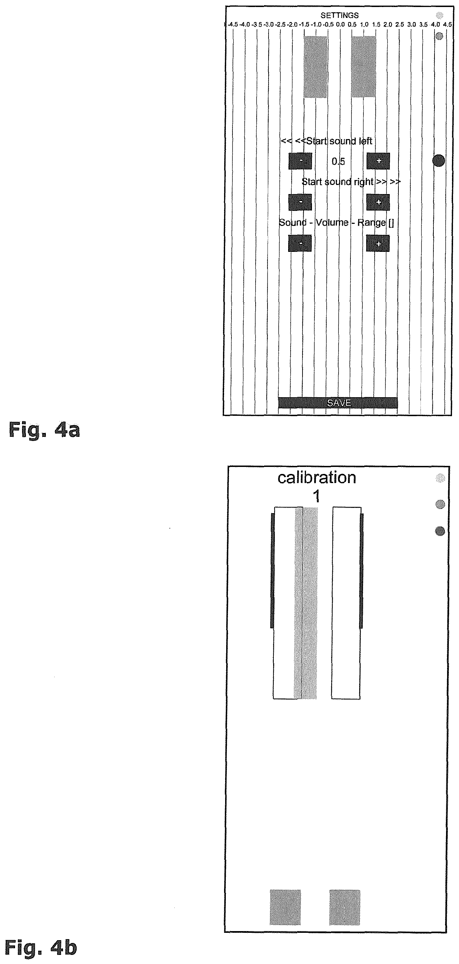

In den Fig. 4a bis 4e sind die verschiedenen Bedieneinheiten und/oder Anzeigeeinheiten des Systems dargestellt, wie sie im Zusammenhang mit unterschiedlichen Verfahrensschritten vorliegen.

Zum Sound bzw. zum akustischen Signal sowie der Neigung ist Folgendes auszuführen: je nach Therapieempfehlung oder eigenem Empfinden kann der Beginn des ansteigenden Tones bei Neigungsgrad 1 und die Maximallautstärke bei Neigungsgrade 2 pro Richtung eingestellt werden (siehe Fig. 4a). So kann entweder ein schnell reagierendes Signal oder eine weich anlaufende akustische Steigerung pro Ohr erzeugt werden. Die Gesamtlautstärke kann vorzugsweise separat eingestellt werden.

In Fig. 4b wird der Kalibrierungsschritt sowie der Start des erfindungsgemäßen Verfahrens dargestellt. Zur Kalibrierung und zum Start ist Folgendes auszuführen: vor Beginn jeder Nutzung, wobei dies sowohl die Nutzung mit Aufzeichnung und die Nutzung ohne Aufzeichnung betrifft, kann ein Kalibrierungsvorgang durchgeführt werden. Der Kalibrierungsvorgang bzw. Kalibrierungsschritt kann beispielsweise fünf Sekunden dauern.

Dadurch ist es möglich, dass auch Personen mit schiefer Grundhaltung das System/das Verfahren/die App nutzen können. Der mindestens eine Lagesensor 20/40 kann schnell am Körper 10 arretiert werden, wobei dieser vom Benutzer wohl nie 100 %ig senkrecht angebracht wird und im vorliegenden Fall auch nicht 100 %ig senkrecht angebracht werden muss.

Während des Kalibrierungsvorgangs kann sich der Benutzer so gerade hinstellen, wie er sich am wohlsten fühlt. Es wird ein Mittelwert ermittelt, wobei dieser anschließend als Neigungsgrad 0 definiert wird.

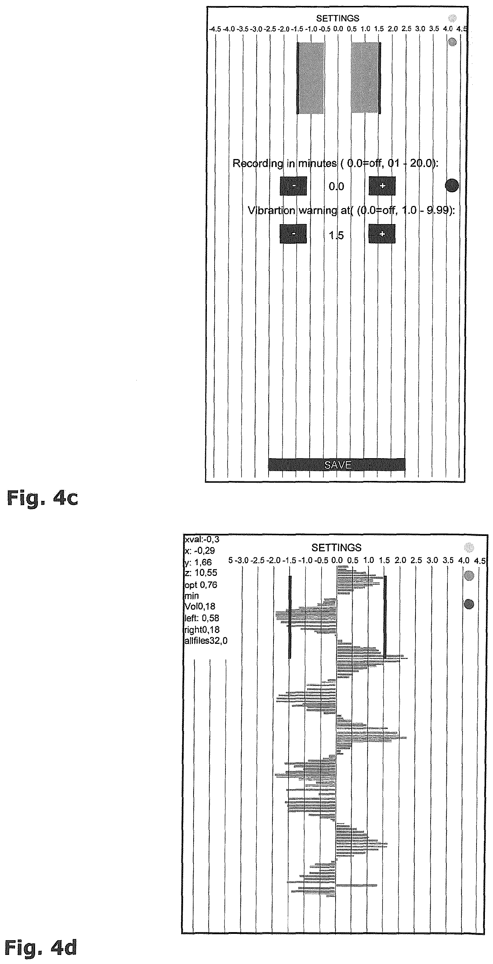

Auch die Möglichkeit der Ausgabe eines Vibrationssignals wird dargestellt. Zur Vibration als Warnsignal zu akustischen Signalen in der Applikation ist Folgendes auszuführen: als Warnanzeige oder für therapeutische Maßnahmen kann in der Applikation pro Neigungsrichtung ein maximaler Neigungswert definiert werden. Wird dieser überschritten, wird vorzugsweise an der Körpermitte ein Vibrationssignal ausgegeben (Fig. 4c).

Zur Vibration und Neigung bei alternativen oder externen Sensoren/Vorrichtungen wird folgendes ausgeführt: bei der Nutzung von externen Bluetooth-Vibrationsgebern, werden diese vorzugsweise an den Schultern angeordnet bzw. den Schultern zugeordnet (siehe Fig. 1b). Diese Vibrationssignale können die Funktion ansteigender akustischer Signale ersetzen, falls die Ohren des Patienten als Feedback-Empfänger ungeeignet sind.

Alternativ oder zusätzlich ist es möglich, einen Vibrationsgeber an der Schläfe einer Person oder an einem Brillengestell anzubringen.

Zu einem Analyse-Aufzeichnungsmodus und dem Speichern in einer Datenbank wird folgendes ausgeführt: Wird der Analysemodus (siehe Fig. 4d) gewählt, werden die Bewegungsdaten des Benutzers in einer lokalen Datenbank aufgezeichnet und visualisiert. Des Weiteren ist es möglich, dass die Aufzeichnung in einer externen Datenbank, beispielsweise cloudbasiert, erfolgen kann. Dies kann angelehnt an eine Wasserwagen-Ansicht von oben (Dosenlibelle) erfolgen (siehe Fig. 3a). Es ist auch möglich, vom Benutzer manuell erstellte Notizen, wie z.B. die Medikamentation, und/oder automatisch erfassbare Daten, wie z.B. GPS-Signale, Wetterdaten etc. zu speichern und/oder an eine Datenbank zu senden.

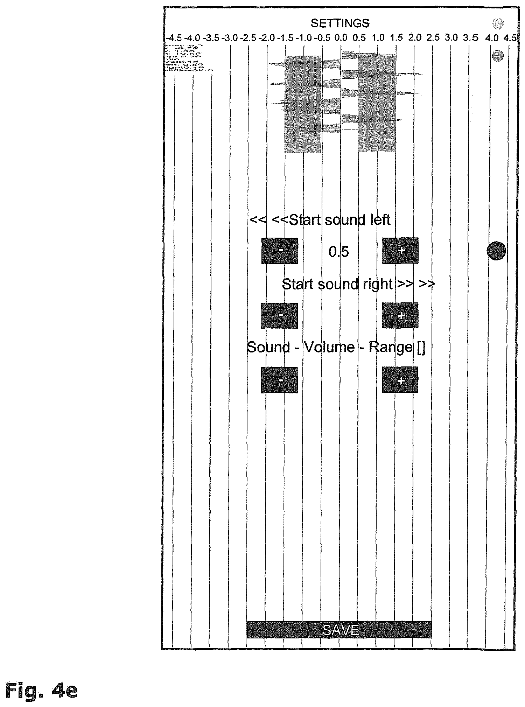

Optional können die Daten über eine Online-Datenbank Ärzten oder Forschern oder Therapeuten oder Trainern zur Verfügung gestellt werden (siehe Fig. 4d). Es wird vorzugsweise eine Analysegraphik erstellt und im Setting-Fenster (siehe Fig. 4a) als Hintergrund eingesetzt, die dem Nutzer für weitere Optimierungen als visuelle Hilfe dienen soll (siehe Fig. 4e).

Prinzipiell gilt, dass akustische Signale via Kopfhörer, die beispielsweise mittels Kabel oder Bluetooth mit dem Smartphone verbunden sind, wiedergegeben werden.

Zum damit erzielten akustischen Feedback ist Folgendes auszuführen: Durch einen zum Neigungsgrad ansteigenden Ton im jeweiligen Ohr, mittels sich ändernder Lautstärke (links, rechts) und Stereo-Toncharakter (vorne, hinten), wird dem Benutzer akustisch in Echtzeit vermittelt, in welche Richtung und wie stark er sich gerade neigt.

Je nach persönlichem Empfinden oder Therapieziel kann das Signal einen Druck-Widerstand oder eine Sogwirkung beim Benutzer erzeugen.

Zum Druck-Widerstand ist Folgendes auszuführen: Ein tiefer Ton steigt über die Lautstärke in dem Ohr an, zu dessen Seite sich auch der Körper neigt. Das heißt: Neigung des Körpers nach rechts; Ton im rechten Ohr wird lauter. Der Benutzer empfindet dies nach einer Eingewöhnungszeit oftmals als ansteigenden Widerstand (Sicherheit, Stabilität) und steigende Warnung.

Zur Sogwirkung ist Folgendes auszuführen: Ein Ton wird auf dem genau zur Neigungsrichtung entgegengesetzten Ohr eingesetzt. Der Benutzer empfindet das unter Umständen nach einer Eingewöhnungszeit als weiche, angenehme Korrektur, weniger als Warnung.

Optional können die Druck- und Sogwirkung(en) auch für (externe) Vibrationsgeber angewendet werden, wie diese beispielsweise in Fig. 1b dargestellt sind.

Im Zusammenhang mit der Druck- oder Sogwirkung ist noch Folgendes auszuführen: Abhängig vom Empfinden beim Benutzer oder der Therapieempfehlung können die Neigungsrichtung und die akustische Seite des Tons (linkes Ohr, rechtes Ohr) auch vertauscht werden.

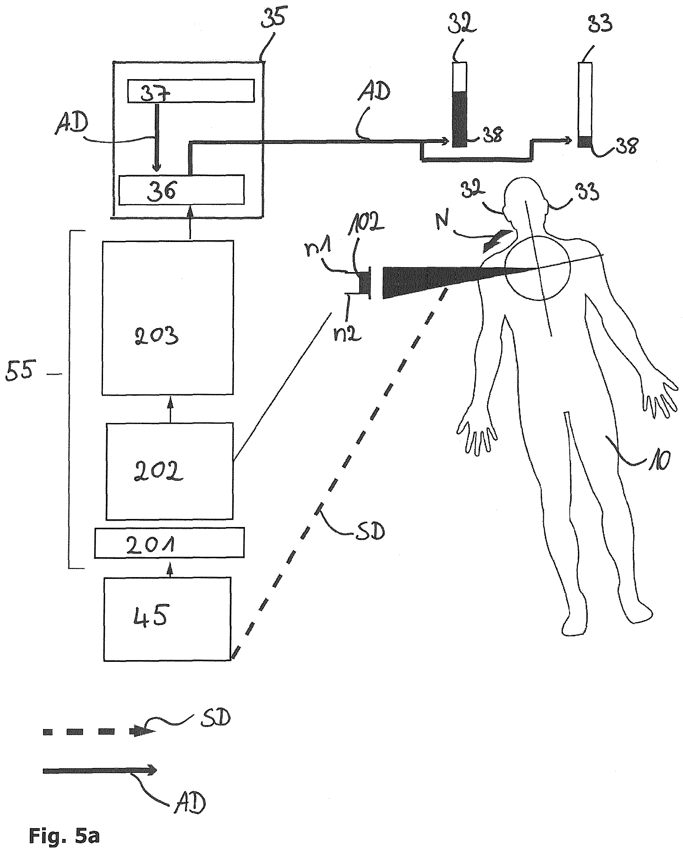

In den Fig. 5a bis 5e werden die erfindungsgemäßen Verfahrensschritte a) bis d) schematisch dargestellt. Insbesondere der Schritt c), nämlich die Umwandlung der Körperlagedaten in mindestens ein akustisches Signal, wird dabei aufgezeigt.

Der Körper 10 weist eine geneigte Körperhaltung auf. Die Neigung bzw. Bewegung wird durch den Pfeil N dargestellt. Ebenfalls zu erkennen sind die Neigungswerte n1 und n2. Diese definieren den aktiven Bereich 102, wie dieser bereits im Zusammenhang mit Fig. 3d erläutert ist. Mit Hilfe einer Körper-Lagesensor-Einheit werden die Körperlagedaten ermittelt. In Fig. 5a ist der Körper 10 in einer Rückansicht dargestellt.

Die Körperlagedaten werden als Sensordaten SD von der Körper-Lagesensor-Einheit 45 an die Recheneinheit 55 gesendet. Dies kann sowohl mittels einer Funkverbindung als auch über ein Kabel erfolgen. Es erfolgt im Schritt 201 die Annahme der Sensordaten. Anschließend, im Schritt 202 werden die empfangenen Sensordaten bzw. Sensorwerte mit den zuvor festgelegten Sensor-Schwellenwerten n1 und n2 verglichen. Im Schritt 203 folgt die Umwandlung der Sensordaten SD, d. h. der Körperlagedaten, in Steuersignale zur Erzeugung eines akustischen Signals.

Dieses Signal wird anschließend an ein Audio-Modul 35 gesendet. Dieses kann sowohl eine Audio-Mixer-Einheit 36 als auch eine Tonerzeugungs-Einheit 37 umfassen. Des Weiteren ist es möglich, dass die Tonerzeugungs-Einheit 37 eine von der Audio-Mixer-Einheit 36 losgelöste Einheit bildet. Die im Schritt 203 in Steuersignale umgewandelten Sensordaten werden an die Audio-Mixer-Einheit gesendet. Zeitgleich kann in der Tonerzeugungs-Einheit 37 ein Stereo-Tonsignal erzeugt werden. Dabei handelt es sich beispielsweise um einen durchgehenden Ton. Alternativ ist das Erzeugen von Tonfolgen oder eines Musikstücks möglich. Dieses Stereo-Tonsignal, insbesondere die Audiodaten AD, kann/können mit Hilfe der Audio-Mixer-Einheit 36 zeitgleich einen linken oder rechten Kanal beeinflussen. Das veränderte Stereo-Signal kann an das linke Ohr 32 sowie an das rechte Ohr 33 beispielsweise durch Kopfhörer weitergeleitet bzw. dort ausgegeben werden.

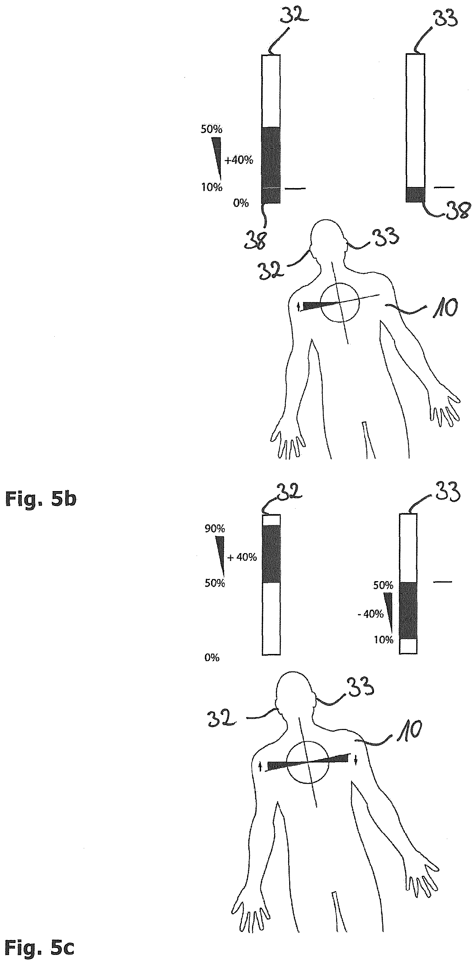

Wie in den Fig. 5b und 5c dargestellt wird, kann ein Basiston 38 an die beiden Ohren 32 und 33 ausgegeben werden. Bei diesem Basiston 38 kann es sich um einen sehr leisen Ton handeln. Der permanente, leise Basiston dient dazu, die erzeugten Tonunterschiede sensitiver wahrnehmen zu können. Außerdem ist der Basiston 38 eine Art Kontroll-Ton. Sofern dieser nicht mehr ausgegeben wird, kann der Benutzer auf eine Störung bzw. Unterbrechung des Verfahrens schließen.

Gemäß der in Fig. 5b dargestellten Methode ist die Lage/Neigung des Körpers nicht zu korrigieren, sofern lediglich der Basiston 38 ausgegeben wird. Ändert sich auf einem Ohr, im vorliegenden Fall auf dem linken Ohr 32, der Ton, beispielsweise hinsichtlich der Lautstärke, der Modulation oder bestimmten Toneffekten, ist die Lage des Körpers zu korrigieren. Aufgrund des auf dem linken Ohr 32 ausgegebenen stereoakustischen Signals wird der Benutzer darüber informiert, dass sein Körper nach links geneigt ist und eine entsprechende Korrekturbewegung nach rechts vorgenommen werden muss.

In Fig. 5c ist eine weitere Möglichkeit hinsichtlich der Umwandlung der Körperlagedaten in ein akustisches Signal dargestellt. Diese Ausführungsform der Umwandlung kann als Stereo-Wechsel-Methode bezeichnet werden. Sofern auf beiden Ohren 32 und 33 ein gleichwertiges Stereosignal, d. h. zu gleichen Anteilen auf dem linken sowie auf dem rechten Ohr zu hören ist, ist die Körperlage bzw. Neigung des Körpers nicht zu korrigieren. Steigert sich auf einer Seite, d. h. in einem Ohr das akustische Signal und wird das akustische Signal zugleich auf dem anderen Ohr gleichwertig reduziert, ist die Lage des Körpers entsprechend zu korrigieren. Im dargestellten Beispiel steigert sich der Ton im linken Ohr 32 ausgehend von 50 % um 40 % auf 90 %. Und auf dem rechten Ohr 33 reduziert sich das akustische Signal ausgehend von 50 % auf 10 %. Dies bedeutet, dass eine Neigung nach links vorliegt und die Körperlage entsprechend nach rechts korrigiert werden muss.

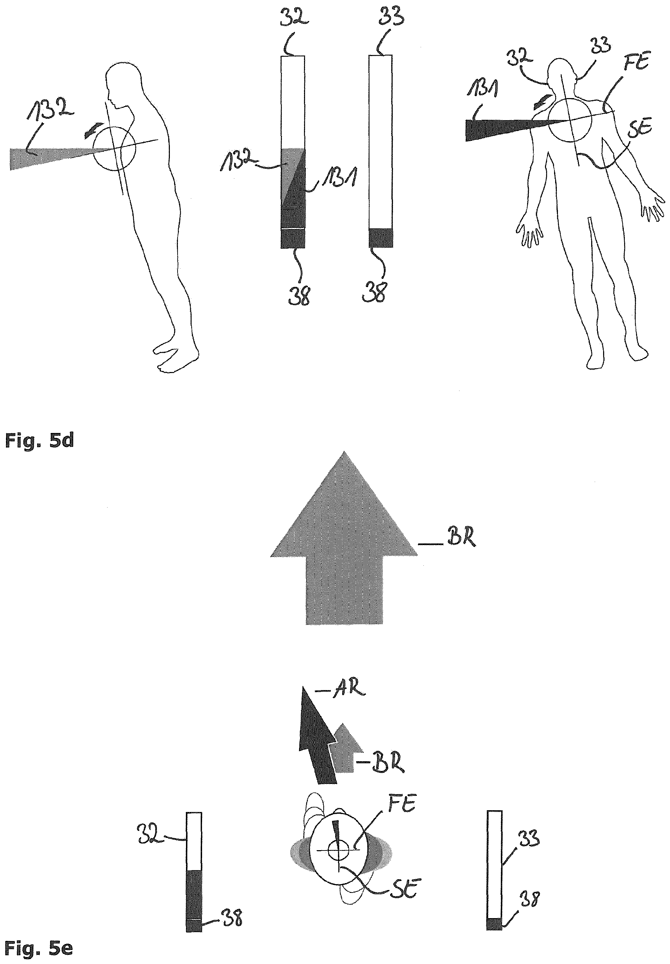

In Fig. 5d wird außerdem angedeutet, dass auch die Neigung in der Sagittalebene bei der Umwandlung der Körperlagedaten in mindestens ein akustisches Signal berücksichtigt wird. Es ist ein erstes akustisches Signal 131 dargestellt. Dieses akustische Signal 131 steht im Zusammenhang mit einer Abweichung von der 0°-Lage des Körpers nach links oder nach rechts.

Es ist weiterhin angedeutet, dass ein zweites akustisches Signal 132 im Zusammenhang mit der Abweichung von der 0°-Lage des Körpers nach vorne oder nach hinten generiert wird. Mit anderen Worten wird bei einer Neigung nach vorne oder nach hinten ein zweites akustisches Signal 132, insbesondere ein zweites stereoakustisches Signal, hinzugefügt. Dieses zweite stereoakustische Signal 132 löst bei einer Neigung nach vorne oder nach hinten das erste stereoakustische Signal 131 je nach Neigungsstärke und vordefinierten Einstellungen zunehmend ab (cross-fade). Dabei richtet sich das zweite akustische Signal 132 aber zeitgleich permanent an das Links-Rechts-Verhältnis des ersten akustischen Signals 131.

Wie im Zusammenhang mit dem linken Ohr 32 dargestellt wird, mischt sich das zweite akustische Signal 132 in Abhängigkeit des Neigungsgrades mit dem ersten akustischen Signal 131. Dies erfolgt bis zur maximalen Übertönung des ersten akustischen Signals 131.

In Fig. 5e wird dargestellt, dass im Schritt a) die durchschnittliche Bewegungsrichtung BR des Körpers 10 und eine Abweichung AR von der durchschnittlichen Bewegungsrichtung bestimmt werden. Auch diese Daten hinsichtlich der Abweichung AR von der durchschnittlichen Bewegungsrichtung BR können im Schritt c), d. h. bei der Umwandlung der Körperlagedaten in mindestens ein akustisches Signal herangezogen werden. Hierbei muss zunächst in einem Verfahrensschritt eine Bewegungsrichtungserkennung erfolgen. Sofern die durchschnittliche Bewegungsrichtung BR bestimmt wurde kann eine Abweichung AR von dieser Bewegungsrichtung BR erkannt bzw. festgestellt werden.

Im dargestellten Beispiel wird die Abweichung AR durch ein akustisches Signal im linken Ohr 32 signalisiert. Somit können auch Abweichungen von einem geradlinigen Gang erkannt und signalisiert werden. Eine Neigung im Oberkörper des Benutzers bzw. des Körpers 10 ist dabei nicht notwendig. Ein Gangbild, das einen Schwank aufweist, steht nicht grundsätzlich im Zusammenhang mit einem schiefen bzw. geneigten Körper. Vielmehr kann eine Schwank-Tendenz durch Ausfall- bzw. Ausgleichsschritte entstehen. Aufgrund der Einbeziehung der Abweichung AR von der Bewegungsrichtung BR bei der Umwandlung der Körperlagedaten in mindestens ein akustisches Signal kann somit eine weiterentwickelte Gangbild-Verbesserung zur Verfügung gestellt werden.

Die Umwandlung der Daten hinsichtlich der Abweichung AR in ein akustisches Signal kann derart erfolgen, wie dies bereits im Zusammenhang mit einer Körperneigung nach links oder rechts (oder nach vorne oder hinten) beschrieben ist. Beispielsweise kann bei Überschreiten eines Schwellenwertes, der auch Minimalwert genannt werden kann, hinsichtlich einer Abweichung von der Bewegungsrichtung nach links im linken Ohr ein akustisches Signal, insbesondere ein ansteigendes akustisches Signal, ausgegeben werden. Eine Abweichung AR von der durchschnittlichen Bewegungsrichtung BR kann beispielsweise durch die Feststellung eines Unterschieds zwischen der durchschnittlichen Bewegungsrichtung BR und der Oberkörperausrichtung erkannt werden.

In Fig. 5e sind des Weiteren die vorher erwähnten Ebenen, nämlich die Sagittalebene SE sowie die Frontalebene FE eingezeichnet.

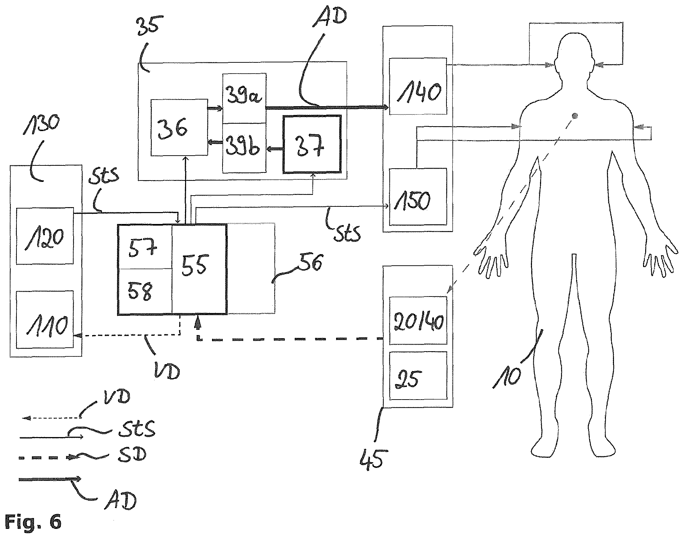

In Fig. 6 werden einzelne Bauteile bzw. Bauteilgruppen des erfindungsgemäßen Systems dargestellt.

Zunächst ist eine Körper-Lagesensor-Einheit 45 dargestellt. Diese Körper-Lagesensor-Einheit 45 kann sowohl Lagesensoren 40 umfassen, die separat von einem mobilen Kommunikationsgerät ausgebildet sind. Des Weiteren kann es sich dabei um Sensoren handeln, die in einem Smartphone 20 integriert sind. Insbesondere kann es sich dabei um Lagesensoren, Gyrosensoren, Gravitationssensoren, Beschleunigungssensoren, Erschütterungssensoren, Rotationssensoren, Magnetsensoren sowie um mechanische Sensorik wie z. B. einen Seilzugsensor handeln.

Des Weiteren kann alternativ oder zusätzlich eine externe Sensoreinheit 25 ausgebildet sein. Dabei kann es sich um eine Kamera, insbesondere um eine Kinect-Kamera, also um eine Kamera mit Tiefensensorik, oder um ein Wackelbrett handeln. Die von der Körper-Lagesensor-Einheit 45 erfassten Körperlagedaten werden als Sensordaten SD anschließend an die Recheneinheit 55 gesendet. Die Recheneinheit 55 kann Teil einer Controller-Einheit 56 sein. Die Controller-Einheit kann des Weiteren einen Speicher 57 umfassen. In dem Speicher 57 kann beispielsweise eine Auswahl von System-Parametern abgelegt sein. Darin können auch die Werte n1 und n2 abgelegt werden. Ebenfalls ist es möglich, die Auswahl der Sound-Methode, Logarithmen, Lautstärkebereiche usw. im Speicher 57 abzulegen.

Die Controller-Einheit 56 kann des Weiteren eine Kommunikations-Einheit 58 aufweisen. Diese Kommunikations-Einheit 58 dient als Schnittstelle zur Datenübermittlung. Dabei können Daten sowohl exportiert als auch importiert werden. Bei der Kommunikations-Einheit 58 kann es sich sowohl um eine USB-Schnittstelle als auch um eine Funkschnittstelle handeln. Mit Hilfe der Kommunikations-Einheit 58 kann des Weiteren ein Internetzugang über UMTS/LTE oder WiFi erfolgen. Des Weiteren kann die Kommunikations-Einheit 58 eine Schnittstelle zu einer Online-Datenbank umfassen.

Des Weiteren kann ein- und Ausgabemodul 130 ausgebildet sein. Dieses kann eine Anzeigeeinheit 110 sowie eine Bedieneinheit 120 umfassen. Mit Hilfe der Bedieneinheit 120 können, wie bereits im Zusammenhang mit Fig. 3d erläutert, Schwellenwerte hinsichtlich der detektierten Sensordaten SD festgelegt werden. Die Anzeigeeinheit 110 dient beispielsweise zur Darstellung von Graphen 101 sowie von aktiven Bereichen 102. Demnach können an die Anzeigeeinheit 110 Videosignale VD gesendet werden.

Ebenfalls dargestellt ist ein Audio-Modul 35. Dieses Audio-Modul 35 kann die bereits beschriebene Audio-Mixer-Einheit 36 sowie die Tonerzeugungs-Einheit 37 umfassen. Das Audio-Modul 35 dient zur Erzeugung von Audiodaten AD, d. h. von akustischen Signalen, die an eine Ausgabeeinheit 140 zur Ausgabe eines akustischen Signals gesendet werden. Die Tonerzeugungs-Einheit 37 ist mit anderen Worten eine Audio-Quelle zur Tonerzeugung. Dabei kann es sich um einen Tongenerator bzw. um einen Synthesizer handeln. Auch ein Audio-Player kann als Tonerzeugungs-Einheit 37 wirken.

Das Audio-Modul 35 kann des Weiteren einen Audio-Signaleingang 39b sowie einen Audio-Signalausgang 39a aufweisen. Ein Audio-Signaleingang 39b ist notwendig, sofern das Audio-Modul 35 eine Tonerzeugungs-Einheit 37 aufweist. Der Audio-Signaleingang 39b kann als Buchse für einen kabelgebundenen Tonempfang sowie als kabelloser Empfänger, z. B. als Bluetooth-Schnittstelle, ausgebildet sein. Der Audio-Signalausgang 39a ist die Schnittstelle des Audio-Moduls 35, die das Audiosignal bzw. die Audiodaten AD an die Ausgabeeinheit zur Ausgabe des akustischen Signals 140 sendet. Dabei kann es sich um eine Buchse für eine kabelgebundene Tonausgabe sowie um einen Sender für die kabellose Tonausgabe, z. B. um eine Bluetooth-Schnittstelle, handeln.

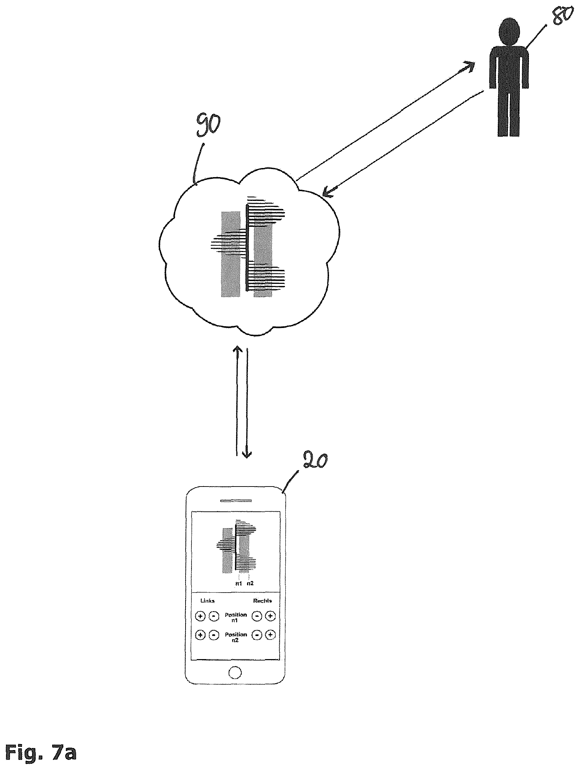

In Fig. 7a ist eine gesicherte Plattform mit Datenbank 90 für Analysedaten dargestellt, die von Forschern, Ärzten 80, Therapeuten und Patienten eingesehen werden kann. Es kann somit eine Verbindung von dem System zu einer Datenbank 90 und von der Datenbank 90 zu einem Arzt 80 hergestellt werden. Vorzugsweise handelt es sich bei der Plattform mit Datenbank 90 um eine Online-Plattform.

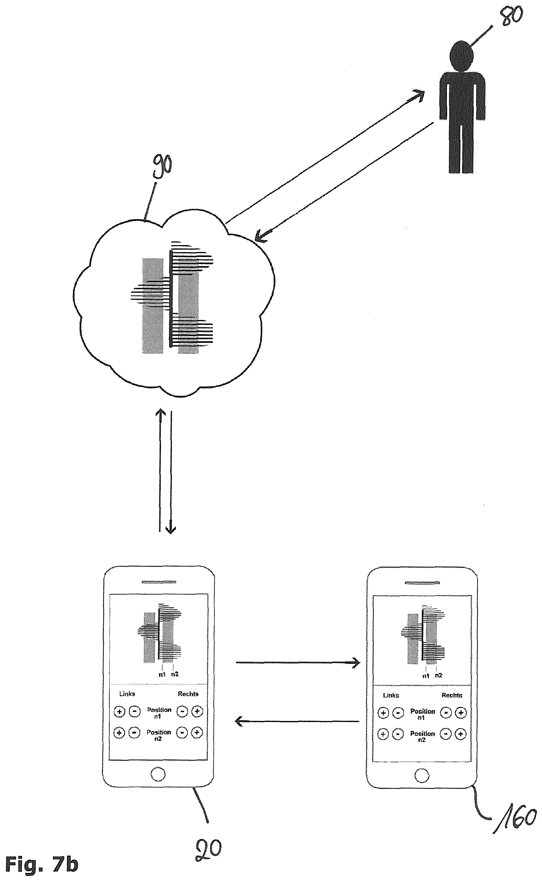

In Fig. 7b ist zusätzlich eine Überwachungseinheit 160 dargestellt. Bei der Überwachungseinheit 160 kann es sich um ein zweites, kabelloses und parallellaufendes Interface-Device, insbesondere um ein mobiles Kommunikationsgerät, handeln, das mit dem System des Benutzers verbunden ist. Die Überwachungseinheit 160 dient dazu, Personen wie Ärzten und Trainern eine Überwachung, aber auch den Eingriff in Echtzeit bei Bedarf zu ermöglichen.

Dies entspricht in etwa einer Remote-Funktion. Hinsichtlich einer Remote-Funktion wird auf Folgendes verwiesen: Für Therapeuten wird ein Echtzeit-Remote-Zugang zum Benutzer ermöglicht, um vor Ort bei Geh-Tests, aber auch mittels Fernanalyse, die Abläufe beobachten und analysieren zu können und optional Anpassungen oder Justierungen an der App/dem Verfahren vornehmen zu können.

Außerdem wird auf weitere Ausführungsformen und/oder Vorteile verwiesen, die mit dem erfindungsgemäßen System und/oder dem erfindungsgemäßen Verfahren und/oder dem erfindungsgemäßen computerlesbaren Speichermedium erzielt werden, verwiesen:
Schnittstellen und Datenanbindungen: Die App/das Verfahren ist für eine unkomplizierte Erweiterbarkeit und Programmierung von Schnittstellen für bestehende Datenbanken vorgesehen. So können die Analysewerte der Patienten nach einer Schnittstellenprogrammierung übermittelt werden.

Externe Vorrichtungen: Alternativ oder zusätzlich zu der App/dem computerlesbaren Speichermedium können eigenständige Sensor- und Feedback-Geräte, insbesondere externe mobile Kommunikationsmittel vorgesehen sein, die den Trage-Komfort, die Akkulaufzeit, die Messgenauigkeit optimieren und in passenden Intervallen mit einem Smartphone oder einem mobilen Kommunikationsgerät verbunden werden und die Messdaten speichern und übermitteln.

So kann modular gewählt, wie eine Ausgabeeinheit zur Ausgabe eines akustischen Signals und eine Ausgabeeinheit zur Ausgabe eines Vibrationssignals kombiniert sind. Die Vorrichtungen kommunizieren selbstständig miteinander.

App für externe Vorrichtung: Für die externen Vorrichtungen kann eine Extra-App ausgebildet sein. Sie verbindet sich per Funksignal, insbesondere per Bluetooth, mit den externen/autarken Vorrichtungen, justiert diese, sammelt Daten ein und macht diese online zugänglich.

Accessoires: Es sind Tragevorrichtungen, insbesondere Taschen und/oder Kleidungsstücke, vorgesehen, die den Trage-Komfort und das Design optimieren. In Fig. 2b ist beispielsweise dargestellt, dass die Tragevorrichtung 60 als ein am Körper 10 enganliegendes Langarm-Shirt ausgebildet sein kann, wobei die Tragevorrichtung 60 mindestens eine Tasche 61 aufweist.

Sound-Entwicklung: Speziell für die jeweiligen Bedürfnisse, Therapieziele und psychologischen Erkenntnisse werden Sounds/Töne zur Verfügung gestellt, die in die App/einen Speicher/ein Programm eingepflegt werden und vorzugsweise online zur Auswahl stehen.

Zielgruppen des erfindungsgemäßen Verfahrens bzw. der erfindungsgemäßen Vorrichtung sind beispielsweise Patienten mit Gleichgewichtsstörungen. Durch die Ergänzung des gestörten Gleichgewichtssinnes mittels zusätzlicher Sensoren, insbesondere mittels zusätzlicher Lagesensoren, besonders bevorzugt mittels einer Körper-Lagesensor-Einheit, und verlässlichem, permanentem und schnell zu bearbeitendem Feedback im Ohr und am Körper entsteht ein Sicherheitsempfinden.

Es kann ein stabiles Selbstbewusstsein des Patienten mit Gleichgewichtsstörungen hervorgerufen werden. Durch den wiederholten Einsatz des Feedbacks bzw. der akustischen Rückmeldung wird durch diese Stimulation eine neue Sensibilisierung der eigenen Körperlage und auch eine erlernte, nachhaltige Körperstabilisierung erzielt.

Das erfindungsgemäße System bzw. das erfindungsgemäße Verfahren umfasst Sicherheits- und Kontrollaspekte für Ärzte, Therapeuten und Patienten in Geh-Therapien bzw. Bewegungstherapien. Es ist eine Korrektur und individuelle Analyse und Betreuung von Menschen mit Gehstörungen, Haltungsstörungen, sowie von Patienten nach Becken- und Knieoperationen in Trainings- oder Rehabilitationsphasen möglich. Durch eine Aufzeichnungsfunktion mit Online-Datenbank können Patienten in Therapiephasen, in Medikation und Behandlung regelmäßig und individuell analysiert und optimiert behandelt werden.

Das erfindungsgemäße System bzw. das erfindungsgemäße Verfahren bietet auch Forschern, Analytikern, Medizinern und Therapeuten Vorteile. Es können Messungen für Analysen mit komfortabler Fortschrittsüberwachung für neue Behandlungsmethoden, Therapie-Sets und Medikamenten an Probanden durchgeführt werden. Auch Sportler und Trainer können von dem erfindungsgemäßen Verfahren bzw. der erfindungsgemäßen Vorrichtung profitieren. Zur Optimierung des Trainings können mittels des Verfahrens, mittels des computerlesbaren Speichermediums, mittels der App oder mittels des Systems Messungen durchgeführt werden. Diese Messungen können auch nach Operationen oder Unfällen durchgeführt werden.

### Bezugszeichenliste

- 10: Körper
- 20: Smartphone
- 25: externe Sensoreinheit
- 30: stereoakustisches Signal
- 31: Kopfhörer
- 32: linkes Ohr
- 33: rechtes Ohr
- 35: Audio-Modul
- 36: Audio-Mixer-Einheit
- 37: Tonerzeugungs-Einheit
- 38: Basiston
- 39a: Audio-Signalausgang
- 39b: Audio-Signaleingang
- 40: Lagesensor
- 45: Körper-Lagesensor-Einheit
- 50: Vibrationssignal
- 55: Recheneinheit
- 56: Controller-Einheit
- 57: Speicher
- 58: Kommunikations-Einheit
- 60: Tragevorrichtung
- 61: Tasche
- 70: Tragevorrichtung
- 71: Tasche
- 72: Brustgurt
- 73: Schultergurt
- 80: Arzt
- 90: Plattform
- 100: Darstellung
- 101: Graph
- 102: aktiver Bereich
- 110: Anzeigeeinheit
- 120: Bedieneinheit
- 130: Ein- und Ausgabemodul
- 131: erstes akustisches Signal
- 132: zweites akustisches Signal
- 140: Ausgabeeinheit zur Ausgabe eines akustischen Signals
- 150: Ausgabeeinheit zur Ausgabe eines Vibrationssignals
- 160: Überwachungseinheit
- 201 bis 203: Verfahrensschritte
- AD: Audiodaten
- AR: Abweichung von Bewegungsrichtung
- BR: Bewegungsrichtung
- FE: Frontalebene
- N: Neigung
- n1: erster Sensorwert
- n2: zweiter Sensorwert
- SD: Sensordaten
- SE: Sagittalebene
- StS: Steuersignal
- VD: Videodaten

## Patentansprüche

1. Verfahren zur Körperhaltungs- und Bewegungsregulation für Menschen, umfassend die Schritte:
a) Bestimmung der Körperlagedaten des menschlichen Körpers (10), insbesondere Bestimmung eines Neigungsgrads des menschlichen Körpers,
b) Senden der Körperlagedaten an eine Recheneinheit (55),
c) Umwandlung der Körperlagedaten in zwei akustische Signale (AD),
d) Senden der akustischen Signale (AD) an eine Ausgabeeinheit (140) nämlich Kopfhörer zur Ausgabe von zwei stereoakustischen Signalen, wobei
im Schritt a) die Körperlagedaten in der Sagittalebene (SE) und in der Frontalebene (FE) des Körpers (10) bestimmt werden und
im Schritt c) ein erstes stereoakustisches Signal (131) für die Körperlagedaten in der Frontalebene (FE) und ein zweites stereoakustisches Signal (132) für die Körperlagedaten in der Sagittalebene (SE) generiert werden,
wobei im Schritt a) Daten hinsichtlich der Schrittkraft erfasst werden und im Schritt c) in die Berechnung der akustischen Signale einfließen.

2. Verfahren nach Anspruch 1,
**dadurch gekennzeichnet, dass**
im Schritt c) ein Vergleich der Körperlagedaten mit Soll-Körperlagedaten durchgeführt wird.

3. Verfahren nach Anspruch 1 oder 2,
**dadurch gekennzeichnet, dass**
vor dem Schritt a) ein Kalibrierungsschritt durchgeführt wird, bei dem die 0°-Lage des menschlichen Körpers (10), insbesondere die 0°-Neigung des menschlichen Körpers, ermittelt wird.

4. Verfahren nach einem der Ansprüche 1 bis 3,
**dadurch gekennzeichnet, dass**
im Schritt a) die durchschnittliche Bewegungsrichtung (BR) des Körpers (10) und eine Abweichung (AR) von der durchschnittlichen Bewegungsrichtung bestimmt werden.

5. Verfahren nach einem der Ansprüche 1 bis 4,
**dadurch gekennzeichnet, dass**
die Körperlagedaten gespeichert werden und/oder die Körperlagedaten an eine Datenbank (90) gesendet werden.

6. Verfahren nach einem der Ansprüche 1 bis 5,
**dadurch gekennzeichnet, dass**
die im Schritt a) bestimmten Körperlagedaten, insbesondere die bestimmten Neigungsgraddaten, in ein visuelles Abbild (100, 101) umgewandelt werden und an eine Anzeigeeinheit (110) gesendet und/oder in einer Datenbank (90) gespeichert werden.

7. System, welches eingerichtet ist, ein Verfahren zur Körperhaltungs- und Bewegungsregulation für Menschen nach einem der Ansprüche 1 bis 6 durchzuführen, umfassend:
- mindestens eine Körper-Lagesensor-Einheit (45),
- mindestens eine Recheneinheit (55), und
- mindestens eine Ausgabeeinheit (140) zur Ausgabe eines akustischen Signals, wobei
die Ausgabeeinheit (140) zur Ausgabe mindestens eines stereoakustischen Signals ausgebildet ist,
wobei zwischen der Recheneinheit (55) und der Ausgabeeinheit (140) zur Ausgabe des stereoakustischen Signals ein Audio-Modul (35) ausgebildet ist, wobei das Audio-Modul (35) mindestens eine Tonerzeugungs-Einheit (37) und mindestens eine Audio-Mixer-Einheit (36) umfasst, wobei die Körper-Lagesensor-Einheit (45) mindestens einen Erschütterungssensor umfasst, wobei
die Ausgabeeinheit (140) ein Kopfhörer (31) ist.

8. System nach Anspruch 7,
**dadurch gekennzeichnet, dass**
die Körper-Lagesensor-Einheit (45) einen Drehbewegungssensor, insbesondere einen Drehratensensor, und/oder einen Gravitationssensor und/oder einen Magnetsensor umfasst.

9. System nach einem der Ansprüche 7 bis 8,
**gekennzeichnet durch**
eine Anzeigeeinheit (110) zur visuellen Darstellung der von der Körper-Lagesensor-Einheit (45) ermittelten Daten.

10. System nach einem der Ansprüche 7 bis 9,
**gekennzeichnet durch**
eine Bedieneinheit (120) zur Einstellung von System-Parametern.

11. System nach einem der Ansprüche 7 bis 10,
**dadurch gekennzeichnet, dass**
die mindestens eine Körper-Lagesensor-Einheit (45) und/oder
die mindestens eine Recheneinheit (55) und/oder
die mindestens eine Ausgabeeinheit (140) zur Ausgabe eines akustischen Signals und/oder
die mindestens eine Anzeigeeinheit (110) und/oder
die mindestens eine Bedieneinheit (120)
in einem mobilen Kommunikationsgerät, insbesondere in einem Mobilfunktelefon (20), oder als Teil eines mobilen Kommunikationsgeräts, insbesondere eines Mobilfunktelefons, ausgebildet ist/sind.

12. System nach einem der Ansprüche 7 bis 11,
**dadurch gekennzeichnet, dass**
die Ausgabeeinheit (140) zur Ausgabe eines stereoakustischen Signals ein ein Knochenleitkopfhörer oder ein Funk-Kopfhörer, insbesondere ein Bluetooth-Kopfhörer, ist.

13. System nach einem der Ansprüche 7 bis 12,
**gekennzeichnet durch**
eine Tragevorrichtung für die mindestens eine Körper-Lagesensor-Einheit (45) und/oder für die mindestens eine Ausgabeeinheit (140) zur Ausgabe eines akustischen Signals und/oder für die mindestens eine Recheneinheit (55) und/oder für die Bedieneinheit (120) und/oder für die Anzeigeeinheit (110), wobei die Tragevorrichtung vorzugsweise als Tasche (71) mit mindestens einem Gurt (72, 73) und/oder als Oberkörper-Bekleidungsstück (60) ausgebildet ist.

14. Computerlesbares Speichermedium, welches Instruktionen enthält, die mindestens einen Prozessor, welcher ein System nach Anspruch 7 steuert, dazu veranlassen, ein Verfahren nach einem der Ansprüche 1 bis 6 zu implementieren, wenn die Instruktionen durch den Prozessor ausgeführt werden.

## Claims

1. A method for posture and movement regulation for humans, said method comprising the steps of:
a) determining the body position data of the human body (10), in particular determining a degree of inclination of the human body,
b) sending the body position data to a computing unit (55),
c) converting the body position data into two acoustic signals (AD),
d) sending the acoustic signal (AD) to an output unit (140), specifically headphones, for outputting two stereo acoustic signals,
wherein
in step a) the body position data are determined in the sagittal plane (SE) and in the frontal plane (FE) of the body (10), and
in step c) a first stereo acoustic signal (131) is generated for the body position data in the frontal plane (FE) and a second stereo acoustic signal (132) is generated for the body position data in the sagittal plane (SE),
wherein in step a) data with respect to the step force are detected and in step c) are included in the calculation of the acoustic signals.

2. The method according to claim 1,
**characterised in that**
in step c) a comparison of the body position data with target body position data is performed.

3. The method according to claim 1 or 2,
**characterised in that**
prior to step a), a calibration step is performed, in which the 0° position of the human body (10), in particular the 0° inclination of the human body, is determined.

4. The method according to any one of claims 1 to 3,
**characterised in that**
in step a) the average direction of movement (BR) of the body (10) and a deviation (AR) from the average direction of movement are determined.

5. The method according to any one of claims 1 to 4,
**characterised in that**
the body position data are stored and/or the body position data are sent to a database (90).

6. The method according to any one of claims 1 to 5,
**characterised in that**
the body position data determined in step a), in particular the determined degree of inclination data, are converted into a visual image (100, 101) and sent to a display unit (110) and/or stored in a database (90) .

7. A system which is designed to carry out a method for posture and movement regulation for humans according to any one of claims 1 to 6, comprising:
- at least one body position sensor unit (45),
- at least one computing unit (55), and
- at least one output unit (140) for outputting an acoustic signal, wherein
the output unit (140) is designed to output at least one stereo acoustic signal,
wherein an audio module (35) is designed to output the stereo acoustic signal between the computing unit (55) and the output unit (140), wherein the audio module (35) comprises at least one sound generation unit (37) and at least one audio mixer unit (36), wherein the body position sensor unit (45) comprises at least one vibration sensor, wherein the output unit (140) is in the form of headphones (31).

8. The system according to claim 7,
**characterised in that**
the body position sensor unit (45) comprises a rotary motion sensor, in particular a rotation rate sensor, and/or a gravitational sensor and/or a magnetic sensor.

9. The system according to any one of claims 7 to 8,
**characterised by**
a display unit (110) for visually displaying the data determined by the body position sensor unit (45).

10. The system according to any one of claims 7 to 9,
**characterised by**
a control unit (120) for setting system parameters.

11. The system according to any one of claims 7 to 10, **characterised in that**
the at least one body position sensor unit (45), and/or
the at least one computing unit (55) and/or
the at least one output unit (140) for outputting an acoustic signal and/or
the at least one display unit (110) and/or
the at least one control unit (120)
is/are formed in a mobile communication device, in particular in a mobile phone (20) or as part of a mobile communication device, in particular of a mobile phone.

12. The system according to any one of claims 7 to 11,
**characterised in that**
the output unit (140) for outputting a stero acoustic signal is formed by bone conduction headphones or radio headphones, in particular Bluetooth headphones.

13. The system according to any one of claims 7 to 12,
**characterised by**
a carrying device for the at least one body position sensor unit (45) and/or for the at least one output unit (140) for outputting an acoustic signal and/or for the at least one computing unit (55) and/or for the control unit (120) and/or for the display unit (110), wherein the carrying device is preferably formed as a pouch (71) with at least one belt (72, 73) and/or as an upper body garment (60).

14. A computer-readable storage medium which contains instructions which prompt at least one processor which controls a system according to claim 7 to implement a method according to any one of claims 1 to 6 when the instructions are executed by the processor.

## Revendications

1. Procédé de régulation de la posture et des mouvements des personnes, comprenant les étapes consistant à :
a) déterminer les données de position du corps humain (10), notamment déterminer un degré d'inclinaison du corps humain,
b) envoyer les données de position du corps à une unité informatique (55),
c) convertir les données de position du corps en deux signaux acoustiques (AD),
d) envoyer les signaux acoustiques (AD) à une unité de sortie (140), à savoir un casque pour émettre deux signaux acoustiques stéréo,
dans lequel
à l'étape a), les données de position du corps dans le plan sagittal (SE) et dans le plan frontal (FE) du corps (10) sont déterminées et
à l'étape c), un premier signal acoustique stéréo (131) pour les données de position du corps dans le plan frontal (FE) et un deuxième signal acoustique stéréo (132) pour les données de position du corps dans le plan sagittal (SE) sont générés,
dans lequel, à l'étape a), des données concernant la force de pas sont enregistrées et à l'étape c) sont incluses dans le calcul des signaux acoustiques.

2. Procédé selon la revendication 1,
**caractérisé en ce que**
à l'étape c) une comparaison des données de position du corps avec des données de position du corps de consigne est effectuée.

3. Procédé selon la revendication 1 ou 2,
**caractérisé en ce que**
à l'étape a) on effectue une étape de calibrage, dans laquelle la position à 0° du corps humain (10), notamment l'inclinaison à 0° du corps humain, est déterminée.

4. Procédé selon une des revendications 1 à 3,
**caractérisé en ce que**
à l'étape a) la direction de mouvement moyenne (BR) du corps (10) et un écart (AR) par rapport à la direction de mouvement moyenne peut être déterminée.

5. Procédé selon une des revendications 1 à 4,
**caractérisé en ce que**
les données de position du corps sont mémorisées et/ou les données de position du corps sont envoyées à une base de données (90).

6. Procédé selon une des revendications 1 à 5,
**caractérisé en ce que**
les données de position du corps déterminées à l'étape a), notamment les données de degré d'inclinaison déterminées, sont converties en une image visuelle (100, 101) et envoyées à une unité d'affichage (110) et/ou mémorisées dans une base de données (90).

7. Système, qui est conçu pour mettre en œuvre un procédé de régulation de posture et de mouvement de personnes selon une des revendications 1 à 6, comprenant :
- au moins une unité de capteur de position du corps (45),
- au moins une unité informatique (55), et
- au moins une unité de sortie (140) pour émettre un signal acoustique, dans lequel
l'unité de sortie (140) est conçue pour émettre au moins un signal acoustique stéréo,
dans lequel entre l'unité informatique (55) et l'unité de sortie (140) pour émettre le signal acoustique stéréo un module audio (35) est réalisé, dans lequel le module audio (35) comprend au moins une unité de génération de son (37) et au moins une unité de mixage audio (36), dans lequel l'unité de capteur de position du corps (45) comprend au moins un capteur de choc, dans lequel l'unité de sortie (140) est un casque (31) .

8. Système selon la revendication 7,
**caractérisé en ce que**
l'unité de capteur de position du corps (45) comprend un capteur de mouvement du corps, notamment un capteur de vitesse de rotation, et/ou un capteur de gravité et/ou un capteur magnétique.

9. Système selon une des revendications 7 à 8,
**caractérisé en ce que**
une unité d'affichage (110) pour une représentation visuelle des données déterminées par l'unité de capteur de position du corps (45).

10. Système selon une des revendications 7 à 9,
**caractérisé par**
une unité de commande (120) pour régler les paramètres du système.

11. Système selon une des revendications 7 à 10,
**caractérisé en ce que**
la au moins une unité de capteur de position du corps (45) et/ou
la au moins une unité informatique (55) et/ou la au moins une unité de sortie (140) pour émettre un signal acoustique et/ou
la au moins une unité d'affichage (110) et/ou la au moins une unité de commande (120) est/sont réalisées dans un appareil de communication mobile, notamment dans un téléphone mobile (20), ou en tant que partie d'un appareil de communication mobile, notamment d'un téléphone mobile.

12. Système selon une des revendications 7 à 11,
**caractérisé en ce que**
l'unité de sortie (140) pour émettre un signal acoustique stéréo est un casque à conduction osseuse ou un casque radio, notamment un casque Bluetooth.

13. Système selon une des revendications 7 à 12,
**caractérisé en ce que**
un dispositif de transport pour au moins une unité de capteur de position du corps (45) et/ou pour au moins une unité de sortie (140) pour émettre un signal acoustique et/ou pour au moins une unité informatique (55) et/ou pour l'unité de commande (120) et/ou pour l'unité d'affichage (110), dans lequel le dispositif de transport est de préférence configuré comme un sac (71) avec au moins une bretelle (72, 73) et/ou comme un vêtement couvrant la partie supérieure du corps (60) .

14. Support de mémorisation lisible par ordinateur, qui contient des instructions, qui amènent au moins un processeur, qui commande un système selon la revendication 7, à mettre en œuvre un procédé selon une des revendications 1 à 6, lorsque les instructions sont exécutées par le processeur.
